# EUROPEAN PATENT APPLICATION

(11) **EP 4 848 293 A1**
(43) Date of publication of application: **02.12.2026**
(21) Application number: 26180732.5
(22) Date of filing: 22.05.2026
(51) Int. Cl.: G16C 20/10, G05B 17/02, G06N 5/022

(54) **GENERATING SYNTHETIC DATA FOR PROCESS ENGINEERING SCHEMATICS OF A CHEMICAL OF INTEREST**

(30) Priority: 29.05.2025 IN 202521052125
(71) Applicant: Tata Consultancy Services Limited, Maharashtra (IN)
(72) Inventor: GUPTA, Shivam, 201306 Noida (IN); SAKHINANA, Sagar Srinivas, 560066 Bangalore (IN); DAS, Akash, 560066 Bangalore (IN); RUNKANA, Venkataramana, 411028 Pune (IN)
(74) Representative: Goddar, Heinz J.

(57) **Abstract**

The disclosure relates generally to methods and systems for generating synthetic data for process engineering schematics of a chemical of interest. Conventional techniques are limited and not effective in auto-generate process flow schematics (e.g., the PFDs) or instrumentation and control layouts (e.g., the PIDs) for new industrial-scale chemical production processes. The methods and systems of the present disclosure discloses a physics-aware Generative Artificial Intelligence (GEN-AI) enriched framework to generate synthetic data from a domain knowledge of chemical process industry. The synthetic data includes a factual instruction-response (IR) dataset, a Direct Preference Optimization (DPO) dataset, a process synthesis, PFD and PID description (SynDIP) dataset, a Logicore dataset, a Retrieval-Augmented Instruction-Tuning (RAIT) dataset. The RAIT dataset further comprises a local RAIT dataset and a global RAIT dataset. The synthetic data is then used to auto-generate the PFDs and the PIDs for the chemical of interest.

## Description

### CROSS-REFERENCE TO RELATED APPLICATIONS AND PRIORITY

The present application claims priority to Indian application no. 202521052125, filed on May 29, 2025.

### TECHNICAL FIELD

The disclosure herein generally relates to chemical process engineering synthesis, and, more particularly, to methods and systems for generating synthetic data for process engineering schematics of a chemical of interest.

### BACKGROUND

Process Flow Diagrams (PFDs) and Piping and Instrumentation Diagrams (PIDs) are standard engineering diagrams used in chemical process industry. A PFD provides a high-level schematic of flow of materials and energy through a chemical production process. The PFDs depict a major equipment, process streams, and key operating conditions for specific units within the chemical production process, without detailing an instrumentation or control systems used during plant operation. The PIDs are built upon the PFDs, offer a more detailed schematic of the instrumentation and the control systems essential for monitoring, operational control, safety, and maintenance of a chemical process plant. The purpose of the PFD is to show what happens in the chemical production process (i.e., key physical or chemical transformations) and where it happens (i.e., in which major equipment units), rather than how the process is controlled or operated. The purpose of the PID is to illustrate how the process operates and is controlled via valves, sensors, control loops, and so on, not just what happens or where it occurs.

Thus, the PFDs and the PIDs serve as foundational documents for chemical process simulations, which in turn drive towards development of digital twins. These digital twins integrate first-principles or data-driven models such as Artificial Intelligence (AI)-based models, with real-time sensor and actuator data, for enabling dynamic monitoring, predictive control and closed-loop process optimization. Further, the data-driven models such as Generative AI (GEN AI) models include but are not limited to large language models (LLMs) and domain specialized small language models (SLMs), help in transforming chemical and materials science by accelerating an autonomous discovery of environmentally sustainable, next-generation specialty chemicals and development of low-cost, high-performance, materials-based products.

The data-driven models for generating the PFDs and the PIDs, are critically dependent on availability of large-scale, high-quality, and appropriately structured training data. However, the chemical process industry faces significant challenges regarding accessibility and suitability of existing data for these purposes. Thus, conventional techniques for auto-generating the PFDs and the PIDs for new industrial-scale chemical production processes, are limited and not effective.

One major limitation is the proprietary and confidential nature of most industrial PFDs and PIDs. These diagrams contain sensitive Intellectual Property (IP) related to process designs and competitive advantages. Consequently, vast repositories of real-world, detailed engineering schematics are not publicly available or easily shareable thus, severely restricting the volume and diversity of accessible data. Furthermore, even when some PFDs or PIDs are found in publicly accessible literature, such as textbooks, academic papers, or older patents, they often suffer from several shortcomings. These publicly available diagrams may be incomplete, simplified, or abstracted for illustrative purposes, lacking intricate details crucial for robust model training. They may not consistently adhere to modem industry standards or reflect the complexity of current industrial processes.

Another major limitation is the lack of structured, machine-readable formats for existing diagrams. Many PFDs and PIDs exist primarily as image files (e.g., scanned Portable Document Formats (PDFs), Computer-Aided Design (CAD) drawings in non-interoperable formats) or embedded figures within the documents. Extracting and converting available visual information from these documents into a structured format suitable for training the data-driven models is a technically complex process, as the training data should not only capture the graphical elements but also the underlying semantic relationships, process logic, instrumentation details, and so on.

### SUMMARY

Embodiments of the present disclosure present technological improvements as solutions to one or more of the above-mentioned technical problems recognized by the inventors in conventional systems.

In an aspect, a processor-implemented method for generating synthetic data for process engineering schematics of a chemical of interest is provided. The method comprising: receiving (i) one or more topics, (ii) one or more seed data, and (iii) a plurality of known chemicals, from a repository, wherein the one or more topics are associated with a domain knowledge of a chemical process industry and the one or more seed data comprises (a) a set of annotated factual instruction-response (IR) pair examples, (b) a set of annotated preference IR pair examples, (c) a set of annotated chemical process blueprints, (d) a set of annotated process flow diagram (PFD) examples, (e) a set of annotated piping and instrumentation diagrams (PID) examples, (f) a set of annotated Logicore IR pair examples, (g) a set of annotated local Retrieval-Augmented Instruction-Tuning (RAIT) IR pair examples, and (h) a set of annotated global RAIT IR pair examples, associated with the domain knowledge of the chemical process industry; generating a plurality of factual IR pairs, from the one or more topics and the one or more seed data, using a first LLM and a multi-metric reward-based filtering, wherein the plurality of factual IR pairs captures domain-specific factual knowledge in the chemical process industry; generating a plurality of direct preference optimization (DPO) IR pairs, from the one or more topics and the one or more seed data, using the first LLM and the multi-metric reward-based filtering, wherein each of the plurality of DPO IR pairs comprises a preferred candidate response and a dispreferred candidate response to an associated DPO instruction; generating a plurality of synDIP IR pairs for each of the plurality of known chemicals, using the first LLM, a second LLM, the one or more seed data, and the multi-metric reward-based filtering, wherein each of the plurality of synDIP IR pairs comprises an industrial synthesis generation textual description, a PFD textual description, and a PID textual description, to an associated SynDIP instruction; generating a plurality of Logicore IR pairs for each of the plurality of known chemicals, using the first LLM, the second LLM, the plurality of synDIP IR pairs, the set of annotated Logicore IR pair examples, and the multi-metric reward-based filtering, wherein each of the plurality of Logicore IR pairs comprises multi-step reasoning with explicit chain-of-thought explanations capturing procedural, logical and engineering response to an associated instruction; generating one or more local Retrieval-Augmented Instruction-Tuning (RAIT) IR pairs for each of the plurality of known chemicals, using the first LLM, the second LLM, the plurality of synDIP IR pairs, the one or more seed data, the multi-metric reward-based filtering, and a Likert-scale-based filtering; generating one or more global RAIT IR pairs, using the first LLM, the second LLM, the plurality of synDIP IR pairs, the one or more seed data, a clustering technique, the multi-metric reward-based filtering, and the Likert-scale-based filtering, wherein the plurality of factual IR pairs, the plurality of DPO IR pairs, the plurality of synDIP IR pairs, the plurality of Logicore IR pairs, the one or more local RAIT IR pairs, and the one or more global RAIT IR pairs form a synthetic dataset; and employing the synthetic dataset to generate a textual description of process engineering schematics of a chemical of interest.

In another aspect, a system for generating synthetic data for process engineering schematics of a chemical of interest is provided. The system includes: a memory storing instructions; one or more Input/Output (I/O) interfaces; and one or more hardware processors coupled to the memory via the one or more I/O interfaces, wherein the one or more hardware processors are configured by the instructions to: receive, (i) one or more topics, (ii) one or more seed data, and (iii) a plurality of known chemicals, from a repository, wherein the one or more topics are associated with a domain knowledge of a chemical process industry and the one or more seed data comprises (a) a set of annotated factual instruction-response (IR) pair examples, (b) a set of annotated preference IR pair examples, (c) a set of annotated chemical process blueprints, (d) a set of annotated process flow diagram (PFD) examples, (e) a set of annotated piping and instrumentation diagrams (PID) examples, (f) a set of annotated Logicore IR pair examples, (g) a set of annotated local Retrieval-Augmented Instruction-Tuning (RAIT) IR pair examples, and (h) a set of annotated global RAIT IR pair examples, associated with the domain knowledge of the chemical process industry; generate a plurality of factual IR pairs, from the one or more topics and the one or more seed data, using a first LLM and a multi-metric reward-based filtering, wherein the plurality of factual IR pairs captures domain-specific factual knowledge in the chemical process industry; generate a plurality of direct preference optimization (DPO) IR pairs, from the one or more topics and the one or more seed data, using the first LLM and the multi-metric reward-based filtering, wherein each of the plurality of DPO IR pairs comprises a preferred candidate response and a dispreferred candidate response to an associated DPO instruction; generate a plurality of synDIP IR pairs for each of the plurality of known chemicals, using the first LLM, a second LLM, the one or more seed data, and the multi-metric reward-based filtering, wherein each of the plurality of synDIP IR pairs comprises an industrial synthesis generation textual description, a PFD textual description, and a PID textual description, to an associated SynDIP instruction; generate a plurality of Logicore IR pairs for each of the plurality of known chemicals, using the first LLM, the second LLM, the plurality of synDIP IR pairs, the set of annotated Logicore IR pair examples, and the multi-metric reward-based filtering, wherein each of the plurality of Logicore IR pairs comprises multi-step reasoning with explicit chain-of-thought explanations capturing procedural, logical and engineering response to an associated instruction; generate one or more local Retrieval-Augmented Instruction-Tuning (RAIT) IR pairs for each of the plurality of known chemicals, using the first LLM, the second LLM, the plurality of synDIP IR pairs, the one or more seed data, the multi-metric reward-based filtering, and a Likert-scale-based filtering; generate one or more global RAIT IR pairs, using the first LLM, the second LLM, the plurality of synDIP IR pairs, the one or more seed data, a clustering technique, the multi-metric reward-based filtering, and the Likert-scale-based filtering, wherein the plurality of factual IR pairs, the plurality of DPO IR pairs, the plurality of synDIP IR pairs, the plurality of Logicore IR pairs, the one or more local RAIT IR pairs, and the one or more global RAIT IR pairs form a synthetic dataset; and employ the synthetic dataset to generate a textual description of process engineering schematics of a chemical of interest.

In yet another aspect, there are provided one or more non-transitory machine-readable information storage mediums comprising one or more instructions which when executed by one or more hardware processors cause: receiving, (i) one or more topics, (ii) one or more seed data, and (iii) a plurality of known chemicals, from a repository, wherein the one or more topics are associated with a domain knowledge of a chemical process industry and the one or more seed data comprises (a) a set of annotated factual instruction-response (IR) pair examples, (b) a set of annotated preference IR pair examples, (c) a set of annotated chemical process blueprints, (d) a set of annotated process flow diagram (PFD) examples, (e) a set of annotated piping and instrumentation diagrams (PID) examples, (f) a set of annotated Logicore IR pair examples, (g) a set of annotated local Retrieval-Augmented Instruction-Tuning (RAIT) IR pair examples, and (h) a set of annotated global RAIT IR pair examples, associated with the domain knowledge of the chemical process industry; generating a plurality of factual IR pairs, from the one or more topics and the one or more seed data, using a first LLM and a multi-metric reward-based filtering, wherein the plurality of factual IR pairs captures domain-specific factual knowledge in the chemical process industry; generating a plurality of direct preference optimization (DPO) IR pairs, from the one or more topics and the one or more seed data, using the first LLM and the multi-metric reward-based filtering, wherein each of the plurality of DPO IR pairs comprises a preferred candidate response and a dispreferred candidate response to an associated DPO instruction; generating a plurality of synDIP IR pairs for each of the plurality of known chemicals, using the first LLM, a second LLM, the one or more seed data, and the multi-metric reward-based filtering, wherein each of the plurality of synDIP IR pairs comprises an industrial synthesis generation textual description, a PFD textual description, and a PID textual description, to an associated SynDIP instruction; generating a plurality of Logicore IR pairs for each of the plurality of known chemicals, using the first LLM, the second LLM, the plurality of synDIP IR pairs, the set of annotated Logicore IR pair examples, and the multi-metric reward-based filtering, wherein each of the plurality of Logicore IR pairs comprises multi-step reasoning with explicit chain-of-thought explanations capturing procedural, logical and engineering response to an associated instruction; generating one or more local Retrieval-Augmented Instruction-Tuning (RAIT) IR pairs for each of the plurality of known chemicals, using the first LLM, the second LLM, the plurality of synDIP IR pairs, the one or more seed data, the multi-metric reward-based filtering, and a Likert-scale-based filtering; generating one or more global RAIT IR pairs, using the first LLM, the second LLM, the plurality of synDIP IR pairs, the one or more seed data, a clustering technique, the multi-metric reward-based filtering, and the Likert-scale-based filtering, wherein the plurality of factual IR pairs, the plurality of DPO IR pairs, the plurality of synDIP IR pairs, the plurality of Logicore IR pairs, the one or more local RAIT IR pairs, and the one or more global RAIT IR pairs form a synthetic dataset; and employ the synthetic dataset to generate a textual description of process engineering schematics of a chemical of interest.

In an embodiment, generating the plurality of factual IR pairs from the one or more topics and the one or more seed data, using the first LLM and the multi-metric reward-based filtering, comprises: decomposing the one or more topics, using the first LLM, to obtain one or more sub-topics for each of the one or more topics; generating a plurality of initial factual IR pairs, from each of the one or more sub-topics associated with each of the one or more topics, using the first LLM, and the set of annotated factual IR pair examples present in the one or more seed data; and applying the multi-metric reward-based filtering on the plurality of initial factual IR pairs, using a pre-defined factual threshold, to generate the plurality of factual IR pairs.

In an embodiment, generating the plurality of DPO IR pairs from the one or more topics and the one or more seed data, using the first LLM and the multi-metric reward-based filtering, comprises: decomposing the one or more topics, using the first LLMs, to obtain one or more sub-topics for each of the one or more topics; generating a plurality of initial DPO IR pairs from each of the one or more sub-topics associated with each of the one or more topics, using the first LLMs and the set of annotated preference IR pair examples present in the one or more seed data, wherein each of the plurality of initial DPO IR pairs comprises a preferred candidate response and a dispreferred candidate response to the associated DPO instruction; and applying the multi-metric reward-based filtering on the plurality of initial DPO IR pairs, using a pre-defined preferred DPO threshold, and a pre-defined dispreferred DPO threshold, to generate the plurality of DPO IR pairs (206c).

In an embodiment, generating the plurality of synDIP IR pairs for each of the plurality of known chemicals, using the first LLM, the second LLM, the one or more seed data, and the multi-metric reward-based filtering, comprises: generating, for each of the plurality of known chemicals, a first industrial synthesis generation textual description and a second industrial synthesis generation textual description, for a predefined industrial synthesis generation template, using the first LLM and the second LLM, respectively, based on the set of annotated chemical process blueprints present in the one or more seed data; aggregating the first industrial synthesis generation textual description and the second industrial synthesis generation textual description, using one of the first LLM and the second LLM, to obtain an aggregated industrial synthesis generation textual description for each of the plurality of known chemicals; generating, for each of the plurality of known chemicals, a first PFD textual description and a second PFD textual description, for a PFD textual description template, based on the aggregated industrial synthesis generation textual description, using the first LLM and the second LLM, respectively, based on the set of annotated PFD examples present in the one or more seed data; aggregating the first PFD textual description and the second PFD textual description, using one of the first LLM and the second LLM, to obtain an aggregated PFD textual description for each of the plurality of known chemicals; generating, for each of the plurality of known chemicals, a first PID textual description and a second PID textual description, for a PID textual description template, based on the aggregated PFD textual description, using the first LLM and the second LLM, respectively, based on the set of annotated PID examples present in the one or more seed data; aggregating the first PID textual description and the second PID textual description, using one of the first LLM and the second LLM, to obtain an aggregated PID textual description for each of the plurality of known chemicals; forming for each of the plurality of known chemicals, a plurality of initial synDIP IR pairs, using the aggregated industrial synthesis generation textual description, the aggregated PFD textual description, and the aggregated PID textual description; and applying the multi-metric reward-based filtering on the plurality of initial synDIP IR pairs, using a pre-defined synDIP threshold, to generate the plurality of synDIP IR pairs for each of the plurality of known chemicals.

In an embodiment, generating the plurality of Logicore IR pairs for each of the plurality of known chemicals, using the first LLM, the second LLM, the plurality of synDIP IR pairs, the set of annotated logicore IR pair examples, and the multi-metric reward-based filtering, comprises: generating, for each of the plurality of known chemicals, a plurality of aggregated Logicore instructions for each of the plurality of synDIP IR pairs, using the first LLM and the second LLM; generating, for each of the plurality of known chemicals, an aggregated Logicore response to each of plurality of aggregated Logicore instructions, using the first LLM, the second LLM, and the set of annotated logicore IR pair examples; forming a plurality of initial Logicore IR pairs for each of the plurality of known chemicals, based on the aggregated Logicore response to each of plurality of aggregated Logicore instructions, from the plurality of synDIP IR pairs; and applying the multi-metric reward-based filtering on the plurality of initial Logicore IR pairs, using a pre-defined Logicore threshold, to generate the plurality of Logicore IR pairs for each of the plurality of known chemicals.

In an embodiment, generating the one or more local RAIT IR pairs for each of the plurality of known chemicals, using the first LLM, the second LLM, the plurality of synDIP IR pairs, the one or more seed data, the multi-metric reward-based filtering, and the Likert-scale-based filtering, comprises: generating a synDIP instruction-response (IR) document for each of the plurality of known chemicals, from the plurality of synDIP IR pairs; extracting a plurality of text chunks for each of the plurality of known chemicals, by parsing the synDIP IR document; generating one or more initial local RAIT IR pairs from each of the plurality of text chunks, using the first LLM, based on the set of annotated local RAIT IR pair examples present in the one or more seed data, for each of the plurality of known chemicals; and applying the multi-metric reward-based filtering and the Likert-scale-based filtering on the one or more initial local RAIT IR pairs generated for each of the plurality of text chunks, using a predefined local RAIT threshold, the second LLM, and a predefined first Likert-scale threshold, to generate the one or more local RAIT IR pairs for each of the plurality of known chemicals.

In an embodiment, generating the one or more global RAIT IR pairs, using the first LLM, the second LLM, the plurality of synDIP IR pairs, the one or more seed data, the clustering technique, the multi-metric reward-based filtering, and the Likert-scale-based filtering, comprises: generating a synDIP IR document for each of the plurality of known chemicals, from the plurality of synDIP IR pairs; extracting a text for each of the plurality of known chemicals, from the synDIP IR document; generating one or more clustered synDIP IR documents, based on the text associated with each of the plurality of known chemicals, using a cosine similarity score; generating one or more initial global RAIT IR pairs from each of the one or more clustered synDIP IR documents, using the first LLM, based on the set of annotated global RAIT IR pair examples present in the one or more seed data; and applying the multi-metric reward-based filtering and the Likert-scale-based filtering on the one or more initial global RAIT IR pairs generated for each of the one or more clustered synDIP IR documents, using a predefined global RAIT threshold, the second LLM, and a predefined second Likert-scale threshold, to generate the one or more global RAIT IR pairs.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 is an exemplary block diagram of a system for generating synthetic data for process engineering schematics of a chemical of interest, in accordance with some embodiments of the present disclosure.
FIGS. 2A and 2B illustrate exemplary flow diagrams of a processor-implemented method for generating synthetic data for process engineering schematics of a chemical of interest, using the system of FIG. 1, in accordance with some embodiments of the present disclosure.
FIG. 3 is a flowchart showing the steps for generating a plurality of factual instruction-response (IR) pairs, from one or more topics and one or more seed data, using a first LLM and a multi-metric reward-based filtering, in accordance with some embodiments of the present disclosure.
FIG. 4 is a flowchart showing the steps for generating a plurality of direct preference optimization (DPO) IR pairs, from the one or more topics and the one or more seed data, using the first LLM and the multi-metric reward-based filtering, in accordance with some embodiments of the present disclosure.
FIGS. 5A and 5B are flowcharts showing the steps for generating a plurality of synDIP IR pairs for each of the plurality of known chemicals, using the first LLM, a second LLM, the one or more seed data, and the multi-metric reward-based filtering, in accordance with some embodiments of the present disclosure.
FIG. 6 is a flowchart showing the steps for generating a plurality of Logicore IR pairs for each of the plurality of known chemicals, using the first LLM, the second LLM, the plurality of synDIP IR pairs, the set of annotated Logicore IR pair examples, and the multi-metric reward-based filtering, in accordance with some embodiments of the present disclosure.
FIG. 7 is a flowchart showing the steps for generating one or more local RAIT IR pairs for each of the plurality of known chemicals, using the first LLM, the second LLM, the plurality of synDIP IR pairs, the one or more seed data, the multi-metric reward-based filtering, and a Likert-scale-based filtering, in accordance with some embodiments of the present disclosure.
FIG. 8 is a flowchart showing the steps for generating one or more global RAIT IR pairs, using the first LLM, the second LLM, the plurality of synDIP IR pairs, the one or more seed data, a clustering technique, the multi-metric reward-based filtering, and the Likert-scale-based filtering, in accordance with some embodiments of the present disclosure.
FIG. 9 is a graph showing evaluation results for a factual IR dataset, in accordance with some embodiments of the present disclosure.
FIGS. 10A and 10B are graphs showing evaluation results for preferred candidate responses and dispreferred candidate responses of a DPO dataset, respectively, in accordance with some embodiments of the present disclosure.
FIG. 11 is a graph showing evaluation results for a SynDIP dataset, in accordance with some embodiments of the present disclosure.
FIG. 12 is a graph showing evaluation results for a Logicore dataset, in accordance with some embodiments of the present disclosure.
FIG. 13 is a graph showing evaluation results for a local RAIT dataset, in accordance with some embodiments of the present disclosure.
FIG. 14 is a graph showing evaluation results for a global RAIT dataset, in accordance with some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The present disclosure solves the technical problems in the art with the methods and systems for generating synthetic data for process engineering schematics of a chemical of interest. The methods and systems of the present disclosure discloses a physics-aware Generative Artificial Intelligence (GEN-AI) enriched framework to generate synthetic data from a domain knowledge of chemical process industry. The synthetic data is then used to auto-generate Process Flow Diagrams (PFDs) and Piping and Instrumentation Diagrams (PIDs) for a new chemical, existing chemical, or a desired chemical of interest, using data-driven models such as Artificial Intelligence (AI) models, and GEN-AI models including but are not limited to large language models (LLMs) and small language models (SLMs).

In accordance with the present disclosure, the synthetic data includes a factual instruction-response (IR) dataset, a Direct Preference Optimization (DPO) dataset, a process synthesis, PFD and PID description (SynDIP) dataset, a Logicore dataset, a Retrieval-Augmented Instruction-Tuning (RAIT) dataset. The RAIT dataset further comprises a local RAIT dataset and a global RAIT dataset. The factual IR dataset includes a plurality of factual instruction-response pairs which capture domain-specific factual knowledge in chemical process engineering to provide the foundational domain knowledge in generating the PFDs and the PIDs for the chemical of interest.

The DPO dataset includes a plurality of DPO IR pairs comprising paired candidate responses labeled as preferred and dispreferred, focusing on aligning the preferences toward more helpful, correct, and contextually appropriate responses. The SynDIP dataset includes a plurality of synDIP IR pairs for each of the plurality of known chemicals, wherein each synDIP IR pair comprises an industrial synthesis generation textual description, a PFD textual description, and a PID textual description.

The Logicore dataset includes a plurality of Logicore IR pairs for each of the plurality of known chemicals, wherein each Logicore IR pair comprises a multi-step reasoning with explicit chain-of-thought explanations capturing procedural, logical, and engineering reasoning steps for analyzing or justifying the PFDs and the PIDs.

The local RAIT dataset includes one or more local RAIT IR pairs which are grounded in semantically coherent intra-document content chunks associated with the domain knowledge of each known chemical. The global RAIT dataset includes one or more global RAIT IR pairs grounded in cross-document semantic clusters supporting multi-source reasoning and synthesis from the domain knowledge of each known chemical combination.

The Logicore dataset, the local RAIT dataset, and the global RAIT dataset are derived from or utilize the SynDIP dataset content (the plurality of synDIP IR pairs) as their primary context. Thus, the Logicore dataset enables reasoning about the content, while the local RAIT dataset grounds responses in specific, small pieces of content and the global RAIT dataset synthesizes the responses from broader, potentially distributed pieces of related content.

Further, the physics-aware Generative Artificial Intelligence (GEN-AI) enriched framework of the present disclosure further employs a multi-metric reward-based filtering and a Likert-scale-based filtering to curate and filter IR pairs while generating the factual IR dataset, the DPO dataset, the SynDIP dataset, the Logicore dataset, and the RAIT dataset. The multi-metric reward-based filtering makes use of metrics such as helpful-ness, correctness, coherence, complexity, and verbosity. The Likert-scale-based filtering works based on the Likert-scale-typically ranging from 1 to 5 (1=Poor, 3=Average, 5=Excellent).

In the context of the present disclosure, a first large language model (LLM) and a second LLMs are chosen from a list of LLMs including but are not limited to OpenAI GPT-4o and Anthropic Claude 3 Haiku.

In the context of the present disclosure, instruction-response (IR) pairs are alternatively referred to as question-answers (QA) pairs. More specifically, each IR or QA pair comprises a response (or an answer) for a given instruction (a question).

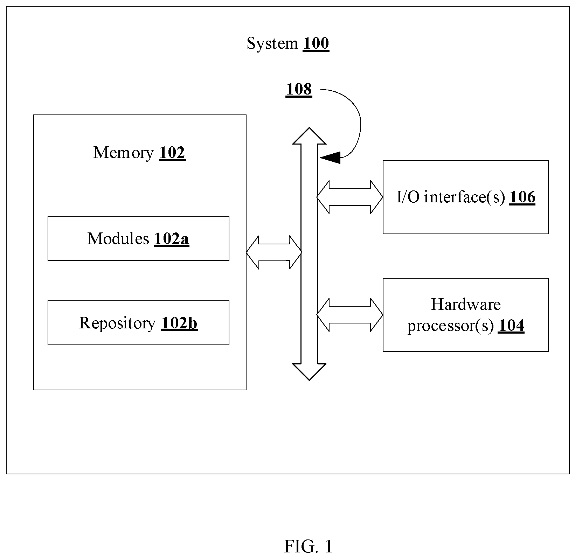

Referring now to the drawings, and more particularly to FIG. 1 through FIG. 14, where similar reference characters denote corresponding features consistently throughout the figures, there are shown preferred embodiments, and these embodiments are described in the context of the following exemplary systems and/or methods.

FIG. 1 is an exemplary block diagram of a system 100 for generating synthetic data for process engineering schematics of a chemical of interest, in accordance with some embodiments of the present disclosure. In an embodiment, the system 100 includes or is otherwise in communication with one or more hardware processors 104, communication interface device(s) or input/output (I/O) interface(s) 106, and one or more data storage devices or memory 102 operatively coupled to the one or more hardware processors 104. The one or more hardware processors 104, the memory 102, and the I/O interface(s) 106 may be coupled to a system bus 108 or a similar mechanism.

The I/O interface(s) 106 may include a variety of software and hardware interfaces, for example, a web interface, a graphical user interface (GUI), and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computers, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules 102a and a repository 102b for storing data processed, received, and generated by one or more of the plurality of modules 102a. The plurality of modules 102a may include routines, programs, objects, components, data structures, and so on, which perform particular tasks or implement particular abstract data types.

The plurality of modules 102a may include programs or computer-readable instructions or coded instructions that supplement applications or functions performed by the system 100. The plurality of modules 102a may also be used as, signal processor(s), state machine(s), logic circuitries, and/or any other device or component that manipulates signals based on operational instructions. Further, the plurality of modules 102a can be used by hardware, by computer-readable instructions executed by the one or more hardware processors 104, or by a combination thereof. In an embodiment, the plurality of modules 102a can include various sub-modules (not shown in FIG. 1). Further, the memory 102 may include information pertaining to input(s)/output(s) of each step performed by the processor(s) 104 of the system 100 and methods of the present disclosure.

The repository 102b may include a database or a data engine. Further, the repository 102b amongst other things, may serve as a database or includes a plurality of databases for storing the data that is processed, received, or generated as a result of the execution of the plurality of modules 102a. Although the repository 102b is shown internal to the system 100, it will be noted that, in alternate embodiments, the repository 102b can also be implemented external to the system 100, where the repository 102b may be stored within an external database (not shown in FIG. 1) communicatively coupled to the system 100. The data contained within such external database may be periodically updated. For example, data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository 102b may be distributed between the system 100 and the external database.

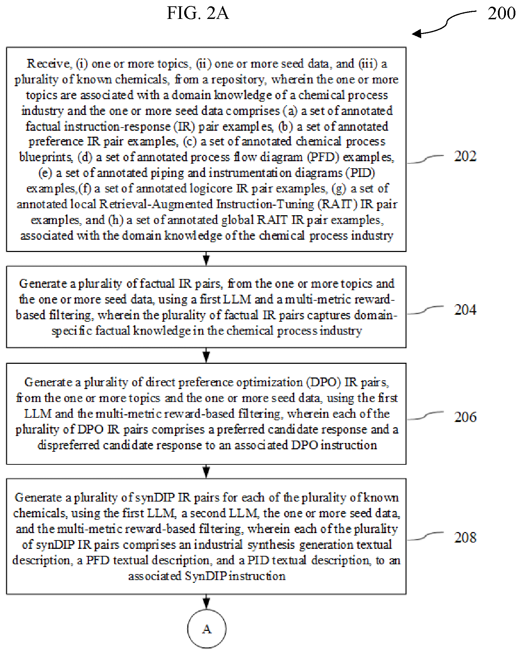

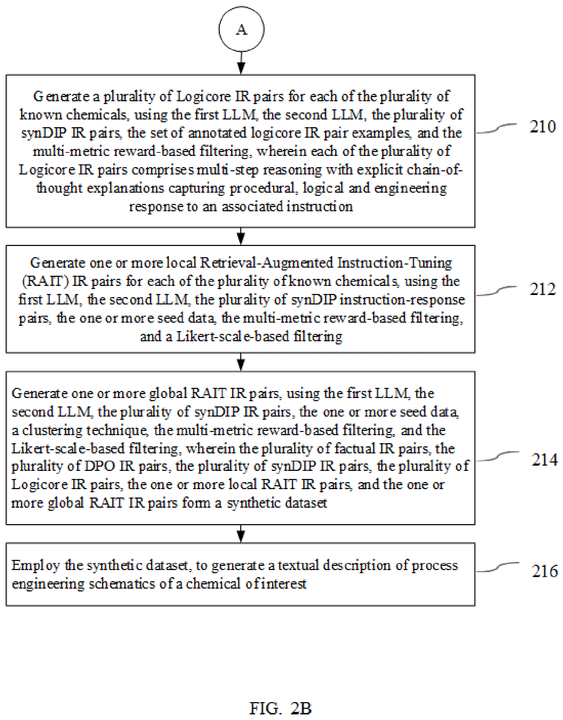

Referring to FIGS. 2A and 2B, components and functionalities of the system 100 are described in accordance with an example embodiment of the present disclosure. For example, FIGS. 2A and 2B illustrate exemplary flow diagrams of a processor-implemented method 300 for generating synthetic data for process engineering schematics of a chemical of interest, using the system 100 of FIG. 1, in accordance with some embodiments of the present disclosure. Although steps of the method 200 including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 202 of the method 200, the one or more input/output (I/O) interfaces 106 of the system 100 are configured to receive one or more topics, one or more seed data, and a plurality of known chemicals, from the repository 102b. The one or more topics are associated with the domain knowledge of a chemical process industry. For example, the one or more topics include but are not limited to heat exchanger design, and fluid mechanics.

The one or more seed data is an annotated data and includes a set of annotated factual IR pair examples, a set of annotated preference IR pair examples, a set of annotated chemical process blueprints, a set of annotated process flow diagram (PFD) examples, a set of annotated piping and instrumentation diagrams (PID) examples, a set of annotated Logicore IR pair examples, a set of annotated local Retrieval-Augmented Instruction-Tuning (RAIT) IR pair examples, and a set of annotated global RAIT IR pair examples, associated with the domain knowledge of the chemical process industry.

The plurality of known chemicals is the list of chemicals that are known in the art, and whose chemical engineering process is already known. For example, some chemicals include but are not limited to Aspirin, Sodium Benzoate, Nitric acid, ethylene, Silicon Dioxide, and Zinc sulfate. In an embodiment, the plurality of known chemicals is curated from a chemical database comprising over 1,120 compounds drawn from different sectors such as electronics, energy storage, pharmaceuticals, advanced manufacturing, and utilities, with a focus on chemicals essential to modem industrial and technological applications.

At step 204 of the method 200, the one or more hardware processors 104 of the system 100 are configured to generate the plurality of factual IR (IR) pairs, from the one or more topics and the one or more seed data received at step 202 of the method 200. The first LLM and the multi-metric reward-based filtering are employed to generate the plurality of factual IR pairs.

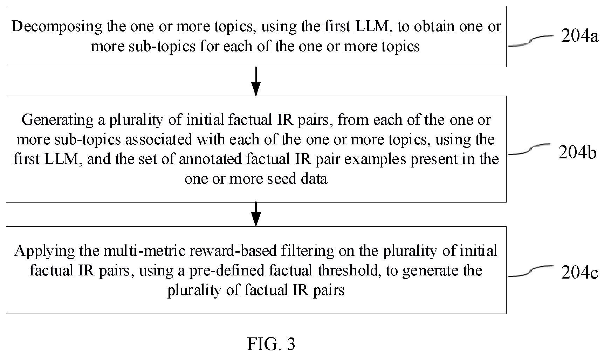

FIG. 3 is a flowchart showing the steps for generating the plurality of factual IR pairs, from the one or more topics and the one or more seed data, using the first LLM and the multi-metric reward-based filtering, in accordance with some embodiments of the present disclosure. As shown in FIG. 3. generating the plurality of factual IR pairs is explained through steps 204a to 204c.

At step 204a, one or more topics are composed using the first LLM, to obtain one or more sub-topics for each of the one or more topics. For example, some of the sub-topics for the topic 'heat exchanger design' include but are not limited to heat transfer concepts, working principle of a heat exchanger, material selection in the heat exchanger, and applications of the heat exchanger. Some of the sub-topics for the topic 'fluid mechanics' include but are not limited to fluid statistics, flow through pipes, Buoyancy and stability, and fluid dynamics.

At step 204b, a plurality of initial factual IR pairs is generated for each of the one or more sub-topics generated at step 204a associated with each of the one or more topics, using the first LLM, and the set of annotated factual IR pair examples present in the one or more seed data. In an embodiment, a predefined prompt is employed to the first LLM to generate the plurality of initial factual IR pairs for each of the one or more sub-topics from the set of annotated factual IR pair examples.

At step 204c, the multi-metric reward-based filtering is applied on the plurality of initial factual IR pairs generated at step 204b, using a pre-defined factual threshold, to generate the factual IR dataset comprising the plurality of factual IR pairs. In an embodiment, the multi-metric reward-based filtering employs a reward model such as a Nemotron-4-340B-Reward model which determines a reward score for each of the plurality of initial factual IR pairs based on the metrics helpfulness, correctness, coherence, complexity, and verbosity. The plurality of initial factual IR pairs whose reward score is more or equal to the pre-defined factual threshold, are then filtered to obtain the plurality of factual IR pairs.

Suppose for a domain-level topic *T* ∈ T (e.g., PFDs or PIDs), where *T* denotes the set of all possible topics. The first LLM decomposes *T* into subtopics *S_{T} =* {*s*₁, ... , *sₙ*}. Then the first LLM M synthesizes the plurality of initial factual (IR) pairs or question-answer (QA) pairs ( *q̃ⱼₖ, ãⱼₖ*) for each sub-topic *sⱼ,* where *j* = 1, ..., n indexes the sub-topics and *k =* 1, ..., *mⱼ* indexes the plurality of initial factual IR pairs within subtopic *sⱼ*. Each of the plurality of initial factual IR pairs is generated as: $\left({{q}^{˜}}_{jk}, {{a}^{˜}}_{jk}\right) ∼ M \left(⋅\left|{s}_{j}, {D}_{seed}^{FQA}\right.\right)$ Here, ${D}_{seed}^{FQA} = {\left\{\left({x}_{i}, {y}_{i}\right)\right\}}_{i = 1}^{N}$ denotes e set of annotated factual IR pair examples present in the one or more seed data. The synthetic initial factual IR pairs form a dataset ${D}_{gen}^{FQA} = {\left\{\left({{q}^{˜}}_{jk}, {{a}^{˜}}_{jk}\right)\right\}}_{j , k}$, which is then filtered via the reward model (e.g., Nemotron-4-340B-Reward), defined as: $R \left({q}^{˜}, {a}^{˜}\right) = {\sum }_{l = 1}^{5} {α}_{l} ⋅ {Metric}_{l} \left({q}^{˜}, {a}^{˜}\right)$ where ${\left\{{Metric}_{l}\right\}}_{l = 1}^{5} = \left\{H, C, {C}_{o}, {C}_{x}, V\right\}$ represent helpful-ness, correctness, coherence, complexity, and verbosity, respectively, and *αₗ* ≥ 0 are predefined scalar weights. Only the initial factual IR pairs satisfying the pre-defined factual threshold (*τ*) (quality threshold) *R*(*q̃, ã*) ≥ *τ* are retained, ensuring the factual IR dataset meets the quality standards required for downstream applications. The resulting factual IR dataset ${D}_{gen}^{FQA}$ contains the plurality of factual IR pairs related to the PFDs and the PIDs.

An exemplary factual IR pair for the topic 'heat exchanger design' is as follows:
" **Instruction** (I)": "How do shell and tube heat exchangers differ from plate heat exchangers?"
"Response (R)": "Shell and tube heat exchangers consist of a series of tubes enclosed in a shell, allowing for high-pressure applications and larger heat transfer areas, while plate heat exchangers use thin plates to create multiple flow channels, offering higher efficiency and easier cleaning in lower-pressure applications."

At step 206 of the method 200, the one or more hardware processors 104 of the system 100 are configured to generate the plurality of DPO IR pairs, from the one or more topics and the one or more seed data. The first LLM and the multi-metric reward-based filtering are employed to generate the plurality of DPO IR pairs. Each of the plurality of DPO IR pairs comprise a preferred candidate response and a dispreferred candidate response to an associated DPO instruction. The preferred candidate response is a candidate response that is preferred for the given instruction or the question, whereas the dispreferred candidate response is the candidate response that is not preferred for the given instruction or the question.

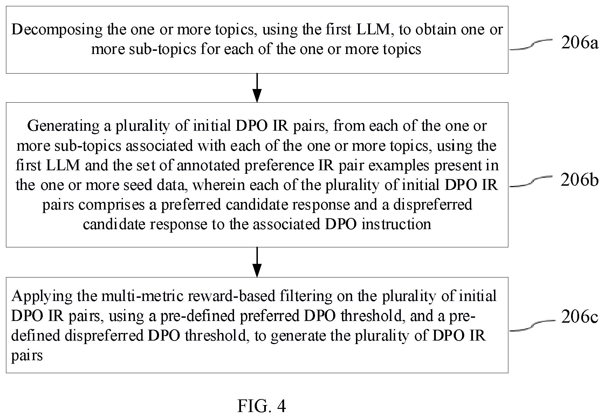

FIG. 4 is a flowchart showing the steps for generating the plurality of DPO IR pairs, from the one or more topics and the one or more seed data, using the first LLM and the multi-metric reward-based filtering, in accordance with some embodiments of the present disclosure. As shown in FIG. 4, generating the plurality of DPO IR pairs is explained through steps 206a to 206c.

At step 206a, the one or more topics received at step 202 are decomposed, using the first LLMs, to obtain one or more sub-topics for each of the one or more topics as mentioned at step 204a. At step 206b, a plurality of initial DPO IR pairs, is generated for each of the one or more sub-topics associated with each of the one or more topics, using the first LLMs and the set of annotated preference IR pair examples present in the one or more seed data. Each of the plurality of initial DPO IR pairs include the preferred candidate response and the dispreferred candidate response to the associated DPO instruction.

At step 206c, the multi-metric reward-based filtering is applied on the plurality of initial DPO IR pairs generated at step 204b, using a pre-defined preferred DPO threshold, and a pre-defined dispreferred DPO threshold, to generate the DPO dataset comprising the plurality of DPO IR pairs. As explained at step 204c, the multi-metric reward-based filtering employs the reward model such as the Nemotron-4-340B-Reward model which determines the reward score for each of the plurality of initial DPO IR pairs based on the metrics helpfulness, correctness, coherence, complexity, and verbosity. The plurality of initial DPO IR pairs with the preferred candidate response and the dispreferred candidate response whose reward score is more or equal to the pre-defined preferred DPO threshold, and the pre-defined dispreferred DPO threshold are then filtered to obtain the plurality of DPO IR pairs.

Suppose for each subtopic *sⱼ* ∈ *S_{T}* (derived from a domain-level topic *T)* and each synthetic instruction (question) ${{q}^{˜}}_{jk} ∈ {D}_{gen}^{FQA}$, two candidate responses are generated through the first LLM M as: $\left({{a}^{˜}}_{jk}^{+}, {{a}^{˜}}_{jk}^{-}\right) ∼ M \left(⋅\left|{{q}^{˜}}_{jk}, {D}_{seed}^{DPO}\right.\right)$ Here, ${{a}^{˜}}_{jk}^{+}$ is the preferred candidate response, ${{a}^{˜}}_{jk}^{-}$ is the dispreferred (alternative) candidate response, and ${D}_{seed}^{DPO}$ is the set of annotated preference IR pair examples present in the one or more seed data. The reward model R then computes a preference gap: $Δ {R}_{jk} = R \left({{q}^{˜}}_{jk}, {{a}^{˜}}_{jk}^{+}\right) - R \left({{q}^{˜}}_{jk}, {{a}^{˜}}_{jk}^{-}\right)$ Where R is defined as a weighted sum over five quality metrics: *{H, C, Cₒ, Cₓ, V},* representing helpfulness, correctness, coherence, complexity, and verbosity, respectively. Only preference triplets satisfying the pre-defined preferred DPO threshold (*τ_{DPO}*), and the pre-defined dispreferred DPO threshold (quality thresholds) (*τ_{DPO}*) Δ*Rⱼₖ* ≥ *τ_{DPO}* are retained, forming the DPO dataset ${D}_{gen}^{DPO}$ with the plurality of DPO IR pairs: ${D}_{gen}^{DPO} = \left\{\left({{q}^{˜}}_{jk}, {{a}^{˜}}_{jk}^{+}, {{a}^{˜}}_{jk}^{-}\right)\left|Δ{R}_{jk}\geq {τ}_{DPO}\right.\right\}$

An exemplary DPO IR pair for the topic 'heat exchanger design' is as follows:
" Instruction (I)": "In what scenarios are air-cooled heat exchangers preferred?"
**"Preferred** candidate response (P_R)": "Air-cooled heat exchangers are preferred in scenarios where water is scarce or expensive, in remote locations without water supply, and in applications requiring low maintenance and reduced risk of corrosion."
" Dispreferred candidate response (D_R)": "Air-cooled heat exchangers are preferred mainly in scenarios where water is abundant, as they are designed to work better with liquid cooling systems rather than in dry environments or remote locations.

At step 208 of the method 200, the one or more hardware processors 104 of the system 100 are configured to generate the plurality of synDIP IR pairs for each of the plurality of known chemicals received at step 202 of the method 200. The first LLM, the second LLM, the one or more seed data, and the multi-metric reward-based filtering are employed to generate the plurality of synDIP IR pairs for each known chemical.

Each of the plurality of synDIP IR pairs include the industrial synthesis generation textual description, the PFD textual description, and the PID textual description to an associated SynDIP instruction. The industrial synthesis generation textual description is a structured process context or a chemical process blueprint of the chemical, including background of the process, operational overview, engineering rationale, chemical reactions, reaction mechanisms, and descriptions of unit operations, materials, process flows, and so on.

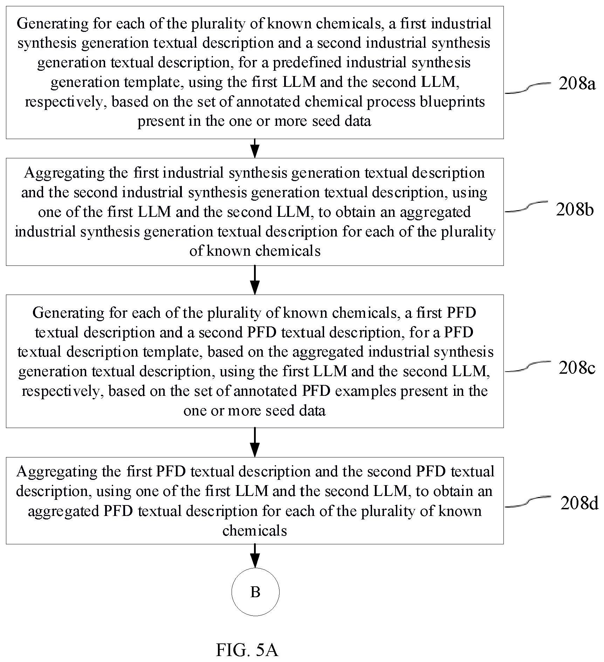

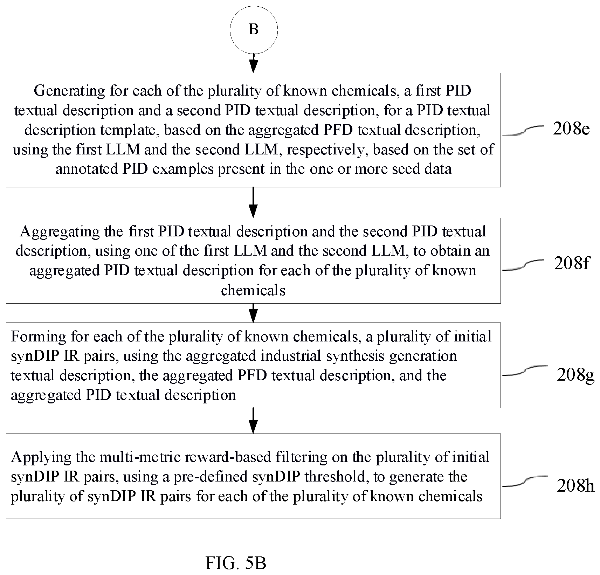

FIGS. 5A and 5B are flowcharts showing the steps for generating the plurality of synDIP IR pairs for each of the plurality of known chemicals, using the first LLM, the second LLM, the one or more seed data, and the multi-metric reward-based filtering, in accordance with some embodiments of the present disclosure. As shown in FIGS. 5A and 5B, generating the plurality of synDIP IR pairs for each known chemical is explained through steps 208a to 208h.

At step 208a, a first industrial synthesis generation textual description and a second industrial synthesis generation textual description, are generated generating for each of the plurality of known chemicals, against a predefined industrial synthesis generation template, using the first LLM and the second LLM, respectively. The set of annotated chemical process blueprints present in the one or more seed data is utilized for generating the first industrial synthesis generation textual description and the second industrial synthesis generation textual description. The first LLM generates the first industrial synthesis generation textual description with the help of the appropriate prompt for the given predefined industrial synthesis generation template of the given known chemical. Similarly, the second LLM generates the second industrial synthesis generation textual description with the help of the same prompt for the same predefined industrial synthesis generation template of the given known chemical.

At step 208b, the first industrial synthesis generation textual description and the second industrial synthesis generation textual description generated at step 208a are aggregated using one of the first LLM and the second LLM, to obtain an aggregated industrial synthesis generation textual description for each of the plurality of known chemicals.

At step 208c, a first PFD textual description and a second PFD textual description are generated for each of the plurality of known chemicals for a PFD textual description template, using the aggregated industrial synthesis generation textual description obtained at step 208b as in input to the first LLM and the second LLM, respectively. The set of annotated PFD examples present in the one or more seed data is utilized for generating the first PFD textual description and the second PFD textual description for each known chemical. The first LLM generates the first PFD textual description with the help of the aggregated industrial synthesis generation textual description against the PFD textual description template. Similarly, the second LLM generates the second PFD textual description with the help of the aggregated industrial synthesis generation textual description against the same PFD textual description template.

At step 208d, the first PFD textual description and the second PFD textual description generated at step 208c are aggregated using one of the first LLM and the second LLM, to obtain an aggregated PFD textual description for each of the plurality of known chemicals.

At step 208e, a first PID textual description and a second PID textual description are generated for each of the plurality of known chemicals for a PID textual description template, based on the aggregated PFD textual description obtained at step 208d, using the first LLM and the second LLM, respectively. The set of annotated PID examples present in the one or more seed data is utilized for generating the first PID textual description and the second PID textual description for each known chemical. The first LLM generates the first PID textual description with the help of the aggregated PFD textual description against the PID textual description template. Similarly, the second LLM generates the second PID textual description with the help of the aggregated PFD textual description against the same PID textual description template.

At step 208f, the first PID textual description and the second PID textual description generated at step 208e, are aggregated using one of the first LLM and the second LLM, to obtain an aggregated PID textual description for each of the plurality of known chemicals.

At step 208g, a plurality of initial synDIP IR pairs is formed for each of the plurality of known chemicals, using the aggregated industrial synthesis generation textual description obtained at step 208b, the aggregated PFD textual description obtained at step 208d, and the aggregated PID textual description obtained at step 208f.

At step 208h, the multi-metric reward-based filtering is applied on the plurality of initial synDIP IR pairs, using a pre-defined synDIP threshold, to generate the SynDIP dataset with the plurality of synDIP IR pairs for each of the plurality of known chemicals. In an embodiment, the multi-metric reward-based filtering employs the reward model such as the Nemotron-4-340B-Reward model which determines the reward score for each of the aggregated industrial synthesis generation textual description obtained at step 208b, the aggregated PFD textual description obtained at step 208d, and the aggregated PID textual description obtained at step 208f.

Then, the aggregated reward score for each of the plurality of initial synDIP IR pairs is calculated by aggregating the reward score of the aggregated industrial synthesis generation textual description obtained at step 208b, the aggregated PFD textual description obtained at step 208d, and the aggregated PID textual description obtained at step 208f. Finally, the plurality of initial synDIP IR pairs whose aggregated reward score is greater than the pre-defined synDIP threshold are retained, to generate the plurality of synDIP IR pairs for each of the plurality of known chemicals.

Suppose for each target chemical, the first LLM M generates the chemical process blueprint *b̃ₖ* in response to the predefined industrial synthesis generation template ${x}_{k}^{SYN}$ (e.g., "Describe a chemical process for producing chemical X, including raw materials, reactions, and equipment"), with: ${{b}^{˜}}_{k} ∼ M \left(⋅\left|{x}_{k}^{SYN}, {D}_{seed}^{SYN}\right.\right)$ Where ${D}_{seed}^{SYN}$ is the set of annotated chemical process blueprints.

Each blueprint *b̃ₖ* is then processed in two stages: (1) the PFD textual description is generated by the first LLM M via the PFD textual description template *x*ₖ*^{PFD}* (using prompt, e.g., "Convert this blueprint to the PFD textual description: [*b̃ₖ*]"), yielding: ${{f}^{˜}}_{k} ∼ M \left(⋅\left|{{x}_{k}}^{PFD}, {{b}^{˜}}_{k}, {D}_{seed}^{PFD}\right.\right)$ Where ${D}_{seed}^{PFD}$ is set of annotated PFD examples; and (2) the PID textual description is generated by the first LLM M using the PID textual description template *x*ₖ*^{PID}* (e.g., "Generate the PID textual description for this PFD textual description: [*f̃*ₖ]"), resulting in: ${{p}^{˜}}_{k} ∼ M \left(⋅\left|{{x}_{k}}^{PID}, {{f}^{˜}}_{k}, {D}_{seed}^{PID}\right.\right)$ Where ${D}_{seed}^{PID}$contains the set of annotated PID examples.
The reward model R of the multi-metric reward-based filtering then evaluates each of the plurality of initial synDIP IR pairs (*xₖ*,*ỹₖ*)-where *ỹₖ* ∈ {*b̃ₖ, f̃*ₖ, *p̃*ₖ} using the metrics *{H, C, Cₒ, Cₓ, V},* (helpfulness, correctness, coherence, complexity, verbosity), and filtered using the aggregated reward score and the pre-defined synDIP threshold (*τ_{SYN}*)*,* using the condition as follows ensuring that each of the plurality of initial synDIP IR pairs includes the validated process context, the PFD description text, and the PID description text for the complete chemical process representation. $R \left({{x}_{k}}^{SYN}, {{b}^{˜}}_{k}\right) + R \left({{x}_{k}}^{PFD}, {{f}^{˜}}_{k}\right) + R \left({{x}_{k}}^{PID}, {{p}^{˜}}_{k}\right) \geq {τ}_{SYN}$ Where *R*(*x*ₖ*^{SYN}*,*b̃*ₖ) represents the reward score of the aggregated industrial synthesis generation textual description, *R*(*x*ₖ*^{PFD}*,*f̃*ₖ) represents the reward score of the aggregated PFD textual description, *R*(*x*ₖ*^{PID}*,*p̃*ₖ) represents the aggregated PID textual description, and the *τ_{SYN}* is the pre-defined synDIP threshold.

An exemplary synDIP IR pair for the chemical 'Acetic Acid' is as follows:
Industrial synthesis generation textual description - Comprehensive Overview of the Industrial Synthesis Process for Acetic Acid\n\n Acetic acid (CH\u2083COOH) is a crucial chemical in various industries, serving as a solvent, a precursor for synthetic fibers, and an intermediate for numerous chemical syntheses. The predominant method for large-scale production of acetic acid is the **methanol carbonylation process**, also known as the **Monsanto process**. This overview provides a detailed synthesis description, focusing on the completeness of chemical reactions, reactor types and conditions, purification steps, by-product handling, energy optimization, and safety measures.\n\n#### 1. Key Chemical Reactions\n\nThe primary reaction for the synthesis of acetic acid via methanol carbonylation is as follows:\n\n**Reaction:**\n\\[ \\text{CH}_3\\text{OH} + \\text{CO} \\rightarrow \\text{CH}_3\\text{COOH} \\]\n\nIn this reaction, methanol (CH\u2083OH) reacts with carbon monoxide (CO) in the presence of a catalyst to produce acetic acid. The reaction is catalyzed by a **rhodium-based catalyst**, often in the form of a rhodium acetate complex, with halide promoters such as methyl iodide (CH\u2083I) to enhance the reaction rate.\n\n**Intermediates:**\n- The reaction proceeds through the formation of an acyl intermediate (CH\u2083C(O)R), which subsequently reacts with methanol to yield acetic acid.\n\n#### 2. Types of Reactors and Operating Conditions\n\n**Reactor Type:**\n- The methanol carbonylation process is typically conducted in a **Continuous Stirred Tank Reactor (CSTR)**, which allows for continuous feed of reactants and removal of products.\n\n**Operating Conditions:**\n- **Temperature:** 150-200 \u00b0C\n- **Pressure:** 30-60 atm\n- The reaction is highly exothermic, necessitating careful temperature control to maintain optimal conditions and prevent thermal degradation of reactants and products.\n\n#### 3. Purification Steps\n\nAfter the reaction, the product mixture contains acetic acid, unreacted methanol, carbon monoxide, and various by-products. The purification process typically involves:\n\n**Distillation:**\n- The crude reaction mixture undergoes a series of distillation steps:\n - **Flash Distillation:** To remove light gases (CO, CH\u2084) and reduce the volume of the feed to the subsequent distillation columns.\n - **Fractional Distillation:** To separate acetic acid from unreacted methanol and other impurities. This is performed in packed or tray columns designed for high separation efficiency.\n - **Azeotropic Distillation:** To remove water and obtain anhydrous acetic acid, ensuring the final product meets industry specifications.\n\n**Equipment Used:**\n- **Flash Drums, Fractionating Columns, and Reboilers:** These are essential for effective separation and purification of acetic acid.\n\n#### 4. Handling and Treatment of By-products and Waste Streams\n\nBy-products from the methanol carbonylation process include:\n- **Methyl Acetate (CH\u2083COOCH\u2083):** This can be recycled back into the carbonylation reactor or sold as a separate product.\n- **Dimethyl Ether (DME):** Can also be recycled or converted into valuable products.\n\n**Waste Treatment:**\n- Waste streams containing unreacted methanol and carbon monoxide are treated using:\n - **Incineration:** To convert hazardous gases into CO\u2082 and H\u2082O.\n - **Scrubbing Systems:** To capture and recycle volatile organic compounds (VOCs).\n\n**Catalyst Recovery:**\n- Catalyst residues are recovered and recycled to minimize waste and improve process efficiency. \n\n#### 5. Heat Integration and Energy Optimization\n\nTo optimize energy use and improve process efficiency, the following systems are integrated:\n\n**Heat Exchangers: * *\n- Heat exchangers are employed to recover heat from the reactor effluent, which can be used to preheat incoming reactants (methanol and CO).\n- Waste heat from distillation columns and other process units is captured and integrated into the plant's steam and power generation systems.\n\n**Energy Recovery:**\n- The exothermic nature of the carbonylation reaction is utilized to drive other process steps, such as distillation, thereby improving overall energy efficiency.\n\n#### 6. Safety Considerations\n\nGiven the hazardous nature of the chemicals involved (methanol, carbon monoxide, and the high-pressure environment), several safety measures are critical:\n\n- **Process Safety Management (PSM):** Implementing a robust PSM program to identify and mitigate risks associated with the process.\n- **Material Safety Data Sheets (MSDS):** Ensuring all personnel are trained and aware of the hazards associated with the chemicals used.\n- **Emergency Shutdown Systems (ESD):** Automated systems to safely shut down operations in case of an emergency.\n- **Ventilation Systems:** Adequate ventilation to prevent the accumulation of toxic gases.\n-**Personal Protective Equipment (PPE):** Operators must wear appropriate PPE, including gloves, goggles, and respirators.\n- **Emergency Response Plans:** Comprehensive plans, including emergency shutdown procedures and spill containment measures, are in place to address unexpected incidents.\n\n### Conclusion\n\nThe industrial synthesis of acetic acid via the methanol carbonylation process is a sophisticated operation that requires careful consideration of chemical reactions, reactor design, purification methods, waste management, energy optimization, and safety protocols. This comprehensive overview serves as a guide for engineers and operators looking to implement or improve acetic acid production in a large-scale industrial setting, ensuring economic viability and environmental sustainability."
**PFD textual description** - Optimized Process Flow Diagram (PFD) Description for the Synthesis of Acetic Acid via Methanol Carbonylation\n\n#### 1. Overview of the Process Flow\n\nThe synthesis of acetic acid from methanol and carbon monoxide is a multi-step process that includes feed preparation, reaction, separation, purification, and waste management. This optimized PFD integrates all major equipment, enhances material flow clarity, identifies potential bottlenecks, and incorporates energy efficiency measures.\n\n#### 2. Major Equipment and Flow of Materials\n\n1. **Feed Preparation**\n - **Equipment:** \n - Methanol Storage Tank (Pressurized)\n - Carbon Monoxide Storage Tank\n - Centrifugal Pump (for Methanol)\n - Multi-stage Compressor (for CO)\n - **Flow:**\n - Methanol (CH\u2083OH) is pumped from the storage tank to the reactor at a flow rate of approximately 1000 kg/h.\n - Carbon monoxide (CO) is compressed to the required pressure (30-60 atm) and supplied to the reactor at a flow rate of approximately 500 kg/h.\n\n2. **Carbonylation Reactor**\n - **Equipment:** Continuous Stirred Tank Reactor (CSTR)\n - **Reaction:**\n \\[ \\text{CH}_3\\text{OH} + \\text{CO} \\rightarrow \\text{CH}_3\\text{COOH} \\]\n - **Operating Conditions:**\n - Temperature: 150-200 \u00b0C\n - Pressure: 30-60 atm\n - **Catalyst:** Rhodium-based catalyst with methyl iodide as a promoter.\n - **Cooling System:** Equipped with a cooling jacket or internal coils to manage exothermic heat.\n\n3. **Reactor Effluent Separation**\n - **Equipment:** \n - Flash Drum\n - Fractional Distillation Column\n - Azeotropic Distillation Column\n - **Flow:**\n - The reactor effluent is sent to a Flash Drum to separate light gases (unreacted CO, CH\u2084) from the liquid stream.\n - The liquid stream is then directed to a Fractional Distillation Column to separate acetic acid from unreacted methanol and impurities.\n - The acetic acid-rich stream undergoes further purification in an Azeotropic Distillation Column to remove water, producing anhydrous acetic acid.\n\n4. **By-product Handling and Recycling**\n - **Equipment:** \n - Separator for Methyl Acetate and Dimethyl Ether\n - **Flow:**\n - Methyl acetate and dimethyl ether by-products are either recycled back to the reactor or processed for sale.\n - Waste streams containing unreacted methanol and CO are treated in an incinerator and scrubber system to convert hazardous gases into CO\u2082 and H\u2082O.\n\n5. **Heat Integration and Energy Optimization**\n - **Equipment:** Heat Exchangers\n - **Integration:**\n - Heat exchangers recover heat from the reactor effluent and distillation columns to preheat incoming methanol and CO feeds.\n - Waste heat is captured for steam generation and other process needs, optimizing overall energy efficiency.\n\n6. **Catalyst Recovery**\n - **Equipment:** Catalyst Recovery Unit\n - **Flow:**\n - Catalyst residues are filtered and returned to the reactor to minimize waste and improve process efficiency.\n\n7. **Safety Considerations**\n - **Systems:**\n - Emergency Shutdown Systems (ESD) for safe operation.\n - Adequate ventilation systems to prevent toxic gas accumulation.\n - Comprehensive emergency response plans, including spill containment measures.\n\n#### 3. Heat Integration and Energy Optimization\n\n- **Heat Recovery:** \n - Heat exchangers are strategically placed to maximize heat recovery from the reactor and distillation units, reducing energy consumption for heating.\n- **Energy Utilization: ** \n - The exothermic nature of the carbonylation reaction is utilized to drive other process steps, such as distillation, enhancing energy efficiency.\n- **Steam Generation:** \n - Recovered heat is used for steam generation, which can be utilized in various process needs, further optimizing energy usage.\n\n#### 4. Potential Bottlenecks and Optimization Suggestions\n\n- **Bottleneck:** Reactor throughput limitations due to catalyst deactivation or insufficient mixing.\n - **Optimization Suggestion:** Implement regular catalyst regeneration and optimize stirring speed for enhanced reaction rates.\n \n-**Bottleneck:** Distillation columns may have limited separation efficiency due to high feed rates.\n - **Optimization Suggestion:** Consider adding trays or packing in the columns to improve separation efficiency.\n\n-**Bottleneck:** Heat exchangers may become fouled, reducing heat transfer efficiency.\n - **Optimization Suggestion:** Establish regular maintenance and cleaning schedules to ensure optimal performance.\n\n#### 5. Conclusion\n\nThis optimized PFD for the synthesis of acetic acid via methanol carbonylation outlines the critical steps, equipment, and considerations necessary for efficient and safe large-scale industrial production. The integration of energy recovery systems, effective recycling of by-products, and robust safety measures ensures economic viability and environmental sustainability in the production process."
**PID textual description** - Optimized Piping and Instrumentation Diagram (P&ID) for Acetic Acid Synthesis via Methanol Carbonylation\n\n#### 1. **Key Components of the P&ID**\n\n- **Equipment Symbols:**\n - **Storage Tanks:** Standard cylindrical symbols with flat tops for Methanol and CO storage.\n - **Centrifugal Pumps:** Indicated with arrows showing flow direction, placed on feed lines.\n - **Carbonylation Reactor:** Continuous Stirred Tank Reactor (CSTR) symbol, prominently displayed.\n - * *Heat Exchangers: ** Standard symbols with flow direction arrows, integrated into the cooling system.\n - **Distillation Columns:** Vertical column symbols with trays indicated, showing feed and product outlets.\n - **Flash Drum:** Flash drum symbol with clear inlet and outlet lines.\n\n#### 2. **Instrumentation and Control Elements**\n\n- **Sensor Placement:**\n - **Temperature Sensors (TT):** \n - At the inlet and outlet of the Carbonylation Reactor.\n - At the Flash Drum and Distillation Column trays.\n - **Pressure Sensors (PT):** \n - At the inlet and outlet of the Carbonylation Reactor.\n - At the Compressor and critical piping sections.\n - **Flow Meters (FT):** \n - On the Methanol and CO feed lines.\n - At the reactor effluent and product lines.\n - **Level Sensors (LT):** \n - In the Methanol and CO Storage Tanks, Flash Drum, and Distillation Column sumps.\n\n- **Control Valves (CV):**\n - **Feed Control Valves (FCV):** \n - On Methanol and CO feed lines, with feedback control loops using flow sensors (FT).\n - **Reactor Outlet Control Valve (PCV):** \n - To manage flow to the Flash Drum, controlled by reactor pressure sensor (PT).\n - **Cooling Control Valve (TCV):** \n - On the reactor cooling system, controlled by reactor temperature sensor (TT).\n - **Level Control Valves (LCV):** \n - On liquid outlets of the Flash Drum and Distillation Columns, controlled by level sensors (LT).\n\n- **Control Loops:**\n - **Feedback Control:** \n - For temperature and pressure in the reactor using PID controllers.\n - **Cascade Control:** \n - For maintaining reactor temperature, where reactor temperature (TT) is the primary control variable, and cooling water flow (TCV) is the secondary control variable.\n\n#### 3. **Safety Instrumentation**\n\n- **Pressure Relief Valves (PRV):** \n - Installed on the Carbonylation Reactor, Flash Drum, and critical piping sections to prevent overpressure.\n- **Emergency Shutdown Systems (ESD):** \n - Manual and automatic shutdown systems with interlocks to isolate the reactor during critical alarms (high pressure, high temperature, low level).\n- **Gas Detectors (GD):** \n - Positioned in the process area to monitor toxic or flammable gases, triggering alarms and emergency response.\n- **Fire and Smoke Detectors (FD, SD):** \n - Throughout the facility, connected to the ESD system and fire suppression systems.\n\n#### 4. **Process Optimization Instrumentation**\n\n- **Advanced Process Control (APC):** \n - Implement APC systems with model predictive control (MPC) algorithms for real-time optimization of reactor performance.\n- **Online Analyzers (AA):** \n - For continuous monitoring of acetic acid concentration and impurity levels in Distillation Column outlets.\n- **Data Historian and Real-Time Analytics:** \n - For collecting, storing, and analyzing process data to identify optimization opportunities.\n\n#### 5. **Redundancy and Reliability**\n\n- **Redundant Sensors:** \n - Critical sensors (temperature, pressure) should have backups to ensure continuous monitoring.\n- **Dual Control Loops:** \n - For critical control parameters, consider dual control loops to enhance reliability.\n- **Maintenance Protocols:** \n - Establish regular maintenance schedules for sensors and control elements to minimize downtime.\n\n#### 6. **Piping Material Recommendations**\n\n- **Material Selection:**\n - **Stainless Steel (316L):** \n - For high-temperature and corrosive environments (reactor, distillation columns).\n - **Carbon Steel:** \n - For low-pressure applications (storage tanks).\n - **PTFE-lined Piping:** \n - For highly corrosive streams to prevent chemical attack.\n\n-**Considerations:**\n - Ensure all materials are compatible with methanol, carbon monoxide, and acetic acid.\n - Evaluate temperature and pressure ratings to ensure safety and integrity.\n\n#### 7. **Control System Integration**\n\n-**Distributed Control System (DCS):** \n - Integrate all sensors, control valves, and actuators into a DCS for centralized monitoring and control.\n-* * Supervisory Control and Data Acquisition (SCADA):** \n - Use SCADA for remote monitoring and control, data logging, and alarm management.\n-**Communication Protocols:** \n - Implement standard communication protocols (e.g., Modbus, Profibus) for seamless integration.\n\n### Conclusion\n\nThis optimized P&ID for the synthesis of acetic acid via methanol carbonylation incorporates detailed instrumentation, control strategies, safety measures, and material considerations. It aligns with industry best practices and standards (e.g., ANSI/ISA-5.1), ensuring a robust and efficient design suitable for large-scale industrial production. The integration of advanced control systems, redundancy measures, and comprehensive safety instrumentation enhances operational reliability and safety, making it a comprehensive solution for acetic acid synthesis."

At step 210 of the method 200, the one or more hardware processors 104 of the system 100 are configured to generate a plurality of Logicore IR for each of the plurality of known chemicals, using the first LLM, the second LLM, the plurality of synDIP IR pairs, the set of annotated Logicore IR pair examples, and the multi-metric reward-based filtering. Each of the plurality of Logicore IR pairs include multi-step reasoning with explicit chain-of-thought explanations capturing procedural, logical and engineering responses to an associated instruction.

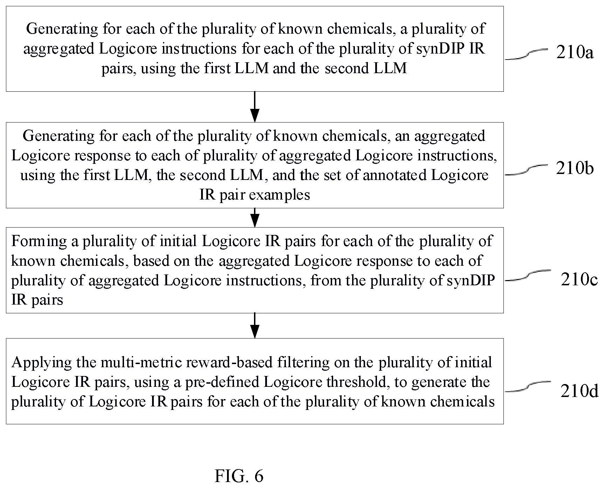

FIG. 6 is a flowchart showing the steps for generating the plurality of Logicore IR pairs for each of the plurality of known chemicals, using the first LLM, the second LLM, the plurality of synDIP IR pairs, the set of annotated Logicore IR pair examples, and the multi-metric reward-based filtering, in accordance with some embodiments of the present disclosure. As shown in FIG. 6, generating the plurality of Logicore IR pairs for each of the plurality of known chemicals is explained through steps 210a to 210d.

At step 210a, for each of the plurality of known chemicals, a plurality of aggregated Logicore instructions is generated for each of the plurality of synDIP IR pairs, using the first LLM and the second LLM. The first LLM generates a plurality of first Logicore instructions and the second LLM generates a plurality of second Logicore instructions. Then both the plurality of first Logicore instructions and the plurality of second Logicore instructions are aggregated to generate the plurality of aggregated Logicore instructions.

At step 210b, for each of the plurality of known chemicals, an aggregated Logicore response is generated to each of plurality of aggregated Logicore instructions obtained at step 210a, using the first LLM, the second LLM, and the set of annotated Logicore IR pair examples. The first LLM generates a first Logicore response for each of plurality of aggregated Logicore instructions and the second LLM generates a second Logicore response for each of plurality of aggregated Logicore instructions, using the set of annotated Logicore IR pair examples. Then the first Logicore response and the second Logicore response are aggregated to obtain the aggregated Logicore response to each of plurality of aggregated Logicore instructions obtained at step 210a.

At step 210c, a plurality of initial Logicore IR pairs is generated for each of the plurality of known chemicals, based on the aggregated Logicore response to each of plurality of aggregated Logicore instructions, from the plurality of synDIP IR pairs.

Then at step 210d, the multi-metric reward-based filtering on the plurality of initial Logicore IR pairs generated at step 210c, using a pre-defined Logicore threshold, to generate the Logicore dataset ( ${D}_{gen}^{LogiCore}$) comprising the plurality of Logicore IR pairs for each of the plurality of known chemicals.

As explained at step 204c, the multi-metric reward-based filtering employs the reward model such as the Nemotron-4-340B-Reward model which determines the reward score for each of the plurality of initial Logicore IR pairs based on the metrics helpfulness, correctness, coherence, complexity, and verbosity. The plurality of initial Logicore IR pairs whose reward score is more or equal to the pre-defined Logicore threshold are then filtered to obtain the plurality of Logicore IR pairs for each of the plurality of known chemicals.

Using the first LLM M, and the set of annotated Logicore IR pair examples ${D}_{seed}^{chem}$ as in ${D}_{gen}^{FQA}$, the logical IR or QA pairs $\left({{q}^{˜}}_{j}, {{a}^{˜}}_{j}\right) ∼ M \left(⋅\left|{x}_{i}, {D}_{seed}^{chem}\right.\right)$ are synthesized, where each *ãⱼ* contains explicit chain-of equipment and controls. For each target chemical, the first LLM M thought reasoning. These pairs are filtered via the established reward model *R*(Nemotron-4-340B-Reward) using the same metrics as in ${D}_{gen}^{FQA} : R \left({{q}^{˜}}_{j}, {{a}^{˜}}_{j}\right) = {Σ}_{l = 1}^{4} {α}_{l} ⋅ {Metric}_{l} \left({{q}^{˜}}_{j}, {{a}^{˜}}_{j}\right) {\left\{{Metric}_{l}\right\}}_{l = 1}^{4} = \left\{H, C, {C}_{o}, {C}_{x}\right\}$ (helpfulness, correctness, coherence, complexity). The plurality of Logicore IR pairs ${D}_{gen}^{LogiCore}$ retains only high-quality reasoning chains, with logical validity implicitly ensured through C (factual alignment with PFD/PID schematics) and *Cₒ* (stepwise flow coherence), maintaining full consistency with the synthetic data generation framework. ${D}_{gen}^{LogiCore} = \left\{\left({{q}^{˜}}_{j}, {{a}^{˜}}_{j}\right)\left|R\left({{q}^{˜}}_{j}, {{a}^{˜}}_{j}\right)\geq {τ}_{logic}\right.\right\}$

An exemplary Logicore IR pair for the chemical 'Acetic acid' is as follows:
" **Instruction** (I)": "What are the key factors influencing the choice of a Continuous Stirred Tank Reactor (CSTR) for the methanol carbonylation process in terms of reaction kinetics and heat management?"
"Response (R)": "The choice of a Continuous Stirred Tank Reactor (CSTR) for the methanol carbonylation process in the synthesis of acetic acid is influenced by several key factors related to reaction kinetics and heat management. Below are the primary considerations:\n\n### 1. **Reaction Kinetics**\n\n- * *Uniform Mixing:* * \n The CSTR provides excellent mixing, ensuring that the reactants (methanol and carbon monoxide) are uniformly distributed throughout the reactor. This uniformity is crucial for maintaining consistent reaction rates and maximizing the conversion of reactants to products.\n\n- **Continuous Operation:** \n The CSTR allows for continuous feeding of reactants and removal of products, which is advantageous for reactions that may have a relatively fast kinetics. This setup helps maintain steady-state conditions, which are essential for optimizing reaction rates and yields.\n\n- **Catalyst Efficiency:** \n The use of a rhodium-based catalyst in the CSTR benefits from the constant agitation, which enhances the contact between the catalyst and reactants. This results in improved catalyst efficiency and reduced catalyst deactivation over time.\n\n### 2. **Heat Management**\n\n- **Exothermic Reaction Control:** \n The methanol carbonylation reaction is highly exothermic, generating significant heat. The CSTR design allows for effective heat management through the incorporation of cooling jackets or internal coils. This enables precise temperature control, which is critical to prevent thermal degradation of reactants and products.\n\n-**Heat Removal:** \n The continuous nature of the CSTR facilitates the removal of heat generated during the reaction. By maintaining optimal operating temperatures (150-200 \u00b0C), the CSTR can prevent local overheating, which could lead to side reactions or reduced product quality.\n\n-**Thermal Stability:** \n The ability to maintain a constant temperature in a CSTR helps ensure that the reaction proceeds at the desired rate without fluctuations that could arise from batch processes. This stability is particularly important for reactions sensitive to temperature changes.\n\n### 3. **Scalability and Flexibility**\n\n- **Scalability:** \n CSTRs are easily scalable, allowing for adjustments in reactor size and throughput to meet production demands. This flexibility is beneficial for industrial applications where production rates may vary.\n\n- **Adaptability to Process Changes:** \n The CSTR can accommodate changes in feed composition or flow rates without significant disruption to the overall process. This adaptability is advantageous in a dynamic production environment.\n\n### Conclusion\n\nIn summary, the choice of a Continuous Stirred Tank Reactor (CSTR) for the methanol carbonylation process is driven by its ability to provide uniform mixing, continuous operation, and effective heat management. These factors collectively enhance reaction kinetics, optimize catalyst performance, and ensure thermal stability, making the CSTR an ideal choice for large-scale acetic acid production."

At step 212 of the method 200, the one or more hardware processors 104 of the system 100 are configured to generate one or more local Retrieval-Augmented Instruction-Tuning (RAIT) IR pairs for each of the plurality of known chemicals. The first LLM, the second LLM, the plurality of synDIP IR pairs, the one or more seed data, the multi-metric reward-based filtering, and a Likert-scale-based filtering are employed to generate the local RAIT IR pairs for each of the plurality of known chemicals.

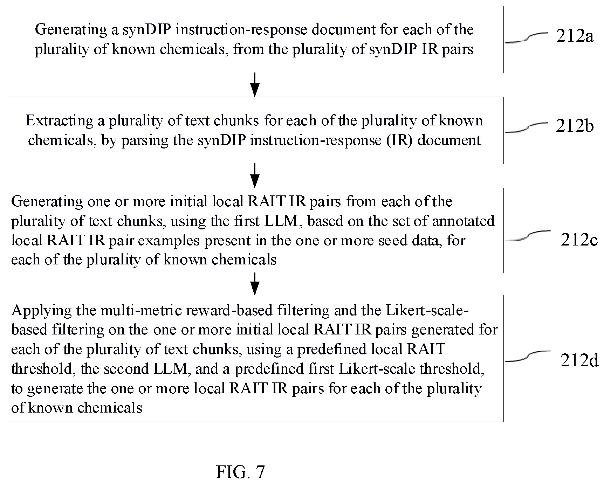

FIG. 7 is a flowchart showing the steps for generating the one or more local RAIT IR pairs for each of the plurality of known chemicals, using the first LLM, the second LLM, the plurality of synDIP IR pairs, the one or more seed data, the multi-metric reward-based filtering, and the Likert-scale-based filtering, in accordance with some embodiments of the present disclosure. As shown in FIG. 7, generating the one or more local RAIT IR pairs for each of the plurality of known chemicals is explained through steps 212a to 212d.

At step 212a, a synDIP IR document is generated for each of the plurality of known chemicals, from the plurality of synDIP IR pairs. The plurality of synDIP IR pairs is copied into a single textual document to obtain the synDIP IR document generated for each of the plurality of known chemicals.

At step 212b, a plurality of text chunks is generated for each of the plurality of known chemicals, by parsing the associated synDIP IR document generated at step 212a. More specifically, the associated synDIP IR document is divided into a textual form to generate the plurality of text chunks for each of the plurality of known chemicals.

At step 212c, one or more initial local RAIT IR pairs are generated from each of the plurality of text chunks obtained at step 212b, using the first LLM, based on the set of annotated local RAIT IR pair examples present in the one or more seed data, for each of the plurality of known chemicals. The first LLM generates the one or more initial local RAIT IR pairs for each known chemical, from each text chunk using the set of annotated local RAIT IR pair examples.

At step 212d, the multi-metric reward-based filtering and the Likert-scale-based filtering are applied on the one or more initial local RAIT IR pairs generated at step 212c for each of the plurality of text chunks. The multi-metric reward-based filtering employs the reward model such as the Nemotron-4-340B-Reward model which determines a first reward score for each of the one or more initial local RAIT IR pairs based on the metrics helpfulness, correctness, coherence, complexity, and verbosity. The second LLM is used to determine a second reward score for each of the one or more initial local RAIT IR pairs. The one or more initial local RAIT IR pairs whose first reward score is more or equal to the predefined local RAIT threshold and the second reward score is more or equal to the predefined first Likert-scale threshold are then filtered to generate the one or more local RAIT IR pairs for each of the plurality of known chemicals.

Unlike ${D}_{gen}^{FQA}$ and ${D}_{gen}^{SynDIP}$, the one or more local RAIT IR pairs integrate retrieval mechanisms to ground the first LLM *M*'s outputs in source documents, mitigating hallucination risks. For each chemical process description from the SynDip dataset associated with each of the plurality of known chemicals (stored as PDF documents containing process flow and instrumentation descriptions), the raw text *T* is extracted and parsed into semantically coherent chunks *C_{T} =* {*c*₁, ... , *cₖ*}, where *cₖ* ~ *Chunk(T)* and each *cₖ* retains contextual continuity with neighboring chunks. The first LLM *M* then synthesizes IR pairs $\left({{q}^{˜}}_{k}, {{a}^{˜}}_{k}\right) ∼ M \left(⋅\left|{c}_{k}, {D}_{seed}^{RAIT}\right.\right)$, conditioned on seed human examples ${D}_{seed}^{RAIT} = \left\{\left({x}_{i}, {y}_{i}\right)\right\}$ that include both instructions and gold-standard retrieval-augmented responses. This approach ensures *q̃ₖ*, *ãₖ* are document-grounded, with *cₖ* providing explicit provenance for generated one or more initial local RAIT IR pairs critical for technical domains where factual alignment with PFD/PID schematics is required.

These one or more initial local RAIT IR pairs are filtered using the same reward model R and Likert-scale-based filtering *L* as ${D}_{gen}^{FQA}$, where $R \left({{q}^{˜}}_{k}, {{a}^{˜}}_{k}\right) = {Σ}_{l = 1}^{5} {α}_{l} ⋅ {Metric}_{l} \left({{q}^{˜}}_{k}, {{a}^{˜}}_{k}\right)$ against *cₖ* and *L*(*q̃ₖ*, *ãₖ*) ∈ {1, ...,5} (1=Poor, 3=Average, 5=Excellent) evaluates answer quality across helpfulness, correctness, and coherence. Only the initial local RAIT IR pairs meeting both thresholds (*τ* for *R,* 4+ for *L)* are retained to generate the local RAIT dataset ( ${D}_{gen}^{LocalRAIT}$) with the one or more local RAIT IR pairs. ${D}_{gen}^{LocalRAIT} = \left\{\left({{q}^{˜}}_{k}, {c}_{k}, {{a}^{˜}}_{k}\right)\left|R\left({{q}^{˜}}_{k}, {{a}^{˜}}_{k}\right)\right.\geq τ∧L\left({{q}^{˜}}_{k}, {{a}^{˜}}_{k}\right)\geq 4\right\}$

An exemplary local RAIT IR pair is as follows:
" **Instruction":** "What type of materials is recommended for piping to handle corrosive materials like acetic anhydride and methanol?"
"Context": "temperatures (D-1, D-2). - **T-5**: Outlet temperature of acetic anhydride (after D-2). - **Pressure Sensors**: -\n**P-1**: Reactor pressure (CSTR). - **P-2**: Separator pressure (S-1). - **P-3**: Distillation column pressures (D-1,\nD-2). - **Flow Meters**: - **FM-1**: Methanol flow rate. - **FM-2**: Acetic acid flow rate. - **FM-3**: Product\nflow rate from distillation. - **Level Sensors**: - **L-1**: Level in the separator (S-1). - **L-2**: Level in the\ncrystallizer (C-1). - **Control Valves**: - **CV-1**: Methanol flow control. - **CV-2**: Acetic acid flow control. -\n**CV-3**: Product flow control from the separator. - **CV-4**: Reflux control in distillation columns. - **CV-5**: Control\nvalve for recycle streams. - **Actuators**: - Electric or pneumatic actuators for control valves to ensure precise control\nbased on feedback from sensors. #### 3. **Control Strategies** - **Feedback Control**: - Utilize temperature and\npressure sensors to maintain optimal conditions in the reactor and distillation columns. - **Feedforward Control**: -\nAdjust feed rates based on upstream conditions to anticipate changes in the reactor. - **Cascade Control**: -\nImplement cascade control for temperature in the reactor, where the outlet temperature is controlled based on the inlet\ntemperature and pressure. #### 4. **Safety Instrumentation** - **Pressure Relief Valves (PRV)**: - **PRV-1**:\nInstalled on the reactor (CSTR). - **PRV-2**: Installed on the separator (S-1). - **PRV-3**: Installed on distillation\ncolumns (D-1, D-2). - **Emergency Shutdown Systems (ESD)**: - **ESD-1**: Automated systems that can isolate the\nreactor and shut down pumps in case of an emergency. - **Interlocks**: - Safety interlocks to prevent operation under\nunsafe conditions (e.g., low level in separators). - **Alarms**: - Visual and audible alarms for high temperature, high\npressure, and low level conditions. #### 5. **Instrumentation for Process Optimization** - **Advanced Process Control\n(APC)**: - Implement APC systems to optimize the operation of the distillation columns and reactor based on real-time\ndata. - **Model Predictive Control (MPC)**: - Utilize MPC for dynamic optimization of the reactor conditions based on\npredictive models. - **Real-Time Data Analytics**: - Integrate data analytics tools to monitor performance and identify\ninefficiencies. #### 6. **Redundancy and Reliability** - **Redundant Sensors**: - Install duplicate temperature and\npressure sensors at critical points to ensure reliability. - **Backup Control Systems**: - Implement backup control\nsystems to maintain operation in case of primary system failure. - **Uninterruptible Power Supply (UPS)**: - Provides\nbackup power to the control systems and critical instrumentation to ensure continuous operation during power outages. \n#### 7. **Piping Materials** - **Material Selection**: - Use stainless steel (e.g., 316L) for piping to handle corrosive\nmaterials like acetic anhydride and methanol. - Consider using Hastelloy or Inconel for high-temperature and\nhigh-pressure applications. #### 8. **Control System Integration** - **Distributed Control System (DCS)**: - Integrate\nall sensors, actuators, and control elements into a DCS for centralized monitoring and control. - **Supervisory Control\nand Data Acquisition (SCADA)**: - Use SCADA for remote monitoring and control, providing operators with real-time\ndata and alerts. ### Conclusion This optimized P&ID for the synthesis of acetic anhydride incorporates critical\ninstrumentation, control strategies, safety measures, and materials selection to ensure efficient and safe operation. By\nadhering to industry best practices and standards, this design is suitable for large-scale industrial production while\noptimizing performance and ensuring compliance with safety regulations. The integration of redundancy and reliability\nfeatures minimizes downtime and enhances operational continuity."
**"Response":** "For piping to handle corrosive materials like acetic anhydride and methanol, it is recommended to use stainless steel (e.g., 316L). Additionally, for high-temperature and high-pressure applications, materials such as Hastelloy or Inconel should be considered."

At step 214 of the method 200, the one or more hardware processors 104 of the system 100 are configured to generate one or more global RAIT IR pairs. The first LLM, the second LLM, the plurality of synDIP IR pairs, the one or more seed data, a clustering technique, the multi-metric reward-based filtering, and the Likert-scale-based filtering are used to generate the one or more global RAIT IR pairs.

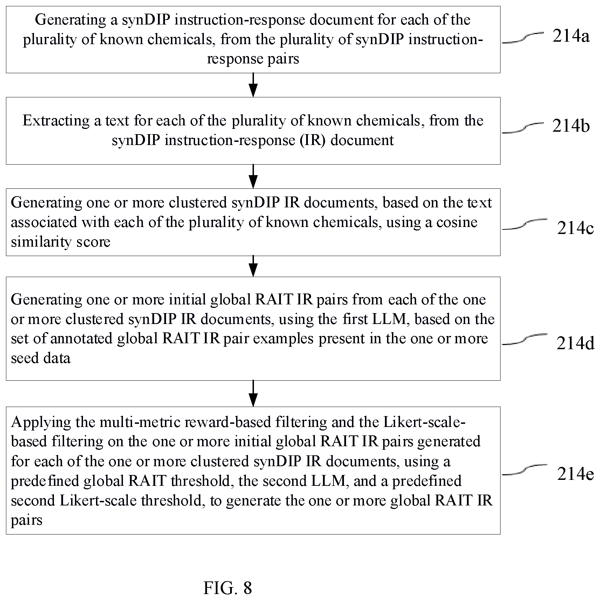

FIG. 8 is a flowchart showing the steps for generating the one or more global RAIT IR pairs, using the first LLM, the second LLM, the plurality of synDIP IR pairs, the one or more seed data, the clustering technique, the multi-metric reward-based filtering, and the Likert-scale-based filtering, in accordance with some embodiments of the present disclosure. As shown in FIG. 8, generating the one or more global RAIT IR pairs is explained through steps 214a to 214e.

At step 214a, a synDIP IR document is generated for each of the plurality of known chemicals, from the plurality of synDIP IR pairs obtained at step 208 of the method 200. The plurality of synDIP IR pairs is copied into a single textual document to obtain the synDIP IR document.

At step 214b, a text for each of the plurality of known chemicals, is extracted from the synDIP IR document obtained at step 214a. At step 214c, one or more clustered synDIP IR documents, based on the text associated with each of the plurality of known chemicals, using a cosine similarity score. The cosine similarity score is determined to analyze the similarity of the text associated with each of the plurality of known chemicals. Then the texts of one or more chemicals, which are similar based on the cosine similarity score are clustered to generate the one or more clustered synDIP IR documents.

At step 214d, one or more initial global RAIT IR pairs are generated from each of the one or more clustered synDIP IR documents, using the first LLM. The set of annotated global RAIT IR pair examples present in the one or more seed data helps in generating the one or more initial global RAIT IR pairs by the first LLM.

Lastly, at step 212e, the multi-metric reward-based filtering and the Likert-scale-based filtering are applied on the one or more initial global RAIT IR pairs generated for each of the one or more clustered synDIP IR documents.

The multi-metric reward-based filtering employs the reward model such as the Nemotron-4-340B-Reward model which determines the first reward score for each of the one or more initial global RAIT IR pairs based on the metrics helpfulness, correctness, coherence, complexity, and verbosity. The second LLM is used to determine the second reward score for each of the one or more initial global RAIT IR pairs. Then, the one or more initial global RAIT IR pairs whose first reward score is more or equal to the predefined global RAIT threshold and the second reward score is more or equal to the predefined second Likert-scale threshold are filtered to generate the one or more global RAIT IR pairs for each of the plurality of known chemicals.

The one or more global RAIT IR pairs present in the global RAIT dataset scales retrieval-augmented generation to both intra- and cross-document comprehension. The chunks *C_{T}* are clustered into semantically related groups *Gⱼ* via cosine similarity *sim*(*φ*(*cᵢ*), *φ*(*cⱼ*)) ≥ *γ*, where *φ* is a domain-tuned embedding model (fine-tuned on T using contrastive learning) optimized for cross-document semantic relationships. For cross-document groups, *Gⱼ* aggregates the chunks from one or more clustered synDIP IR documents. The first LLM M generates multi-scale answers $\left({{a}^{˜}}_{j}^{long}, {{a}^{˜}}_{j}^{short}\right) ∼ M \left(.\left|{G}_{j}, {D}_{seed}^{Global}\right.\right)$, conditioned on the set of annotated global RAIT IR pair examples ${D}_{seed}^{Global}$ that include cross-document seed IR pairs.${D}_{gen}^{GlobalRAIT} = \left\{\left({{q}^{˜}}_{j}, {G}_{j}, {{a}^{˜}}_{j}^{short}\right)|R\left({{a}^{˜}}_{j}^{long}\right)\geq τ∧R\left({{a}^{˜}}_{j}^{short}\right)\geq τ∧L\geq 4\right\}$ Where ${{a}^{˜}}_{j}^{long}$ provides multi-sentence, structured reasoning with evidence and trade-offs, while ${{a}^{˜}}_{j}^{short}$ offers 1-2 sentence surface-level facts. Filtering follows the same criteria as ${D}_{gen}^{SynDIP}$*,* applying both the reward threshold *τ* and a Likert score of *L* ≥ 4. By leveraging grouped chunk clusters, the global RAIT dataset enables the second LLM to generate contextually grounded responses that synthesize information across intra- and inter-document contexts.

An exemplary local RAIT IR pair is as follows:
" **Instruction":** "What are the operating conditions for the oxidative dehydrogenation of n-Butene in the synthesis of 1,3-Butadiene?"
**"Context":** "Synthesis Description\n### Comprehensive Overview of the Industrial Synthesis of 1,3-Butadiene 1,3-Butadiene (C4H6) is a vital intermediate\nin the production of synthetic rubber and various chemicals. Its industrial synthesis can be achieved through multiple\npathways, primarily including the dehydrogenation of butanes and butenes, oxidative dehydrogenation of n-butene, and\ndehydration of ethanol followed by dimerization. This overview provides a detailed synthesis process, including chemical\nreactions, reactor types, operating conditions, purification steps, waste management, energy integration, and safety\nmeasures. #### 1. Key Chemical Reactions **A. Dehydrogenation of Butanes:** - **Reactants:** n-Butane (C4H10) -\n**Reaction:** \\[ \\text{C}_4\\text{H}_{10} \\rightarrow \\text{C}_4\\text{H}_6 + \\text{H}_2 \\] - **Reactor Type:** Fixed-bed\ncatalytic reactor (FBR) - **Catalyst:** Chromium-based or platinum-based catalysts - **Operating Conditions:** -\nTemperature: 550-650 degreesC - Pressure: 1-5 bar **B. Oxidative Dehydrogenation of n-Butene:** - **Reactants:**\nn-Butene (C4H8) - **Reaction:** \\[ \\text{C}_4\\text{H}_8 + \\text{O}_2 \\rightarrow \\text{C}_4\\text{H}_6 +\n\\text{H}_2\\text{O} \\] - **Reactor Type:** Fixed-bed catalytic reactor (FBR) - **Catalyst:** Bismuth molybdate or\nvanadium-based catalysts - **Operating Conditions:** - Temperature: 500-600 degreesC - Pressure: 1-5 bar **C.\nEthanol Dehydration and Dimerization:** - **Reactants:** Ethanol (C2H5OH) - **Reactions:** 1. Dehydration to\nethylene: \\[ \\text{C}_2\\text{H}_5\\text{OH} \\rightarrow \\text{C}_2\\text{H}_4 + \\text{H}_2\\text{O} \\] 2. Dimerization to\n1,3-butadiene: \\[ 2\\text{C} _2\\text{H}_4 \\rightarrow \\text{C}_4\\text{H}_6 \\] - **Reactor Types:** Fixed-bed catalytic\nreactor for dehydration, followed by a separate reactor for dimerization - **Catalysts:** Alumina or silica-alumina for\ndehydration; acidic catalysts (e.g., sulfuric acid) for dimerization - **Operating Conditions:** Varies based on specific\nreactor design and catalyst used. #### 2. Purification Steps The crude product mixture contains various hydrocarbons,\nincluding unreacted feed, hydrogen, and by-products. The purification process typically involves: **A. Distillation:** - \n**Fractional Distillation:** The crude product is fed into a distillation column where components are separated based on\nboiling points. The top fraction is collected as 1,3-butadiene, while heavier fractions are removed from the bottom. -\n**Equipment:** Distillation columns with trays or packing material, operating under reduced pressure to enhance\nseparation efficiency. **B. Stabilization:** - The purified 1,3-butadiene is stabilized by adding inhibitors (e.g.,\ntert-butylcatechol) to prevent polymerization during storage and transportation. **C. Adsorption and Drying:** -\n**Adsorption:** Molecular sieves or activated carbon are used to remove impurities such as sulfur compounds and trace\ncontaminants. - **Drying Agents:** Calcium chloride or molecular sieves are employed to remove moisture from the\nbutadiene stream. #### 3. Handling and Treatment of By-products and Waste Streams By-products from the synthesis\nprocesses may include unreacted hydrocarbons, hydrogen, and heavier olefins. - **Unreacted Hydrocarbons: ** Can be\nrecycled back into the reactor or sent to a separate recovery unit for further processing. - **Hydrogen:** Captured and\nutilized as a fuel source or for other chemical processes. - **Heavy By-products:** May be processed further or sold as\nfeedstock for other chemical processes. **Waste Management:** - **Wastewater Treatment:** Residual organic\ncompounds are removed, and pH is neutralized before discharge or further treatment. - **Flare Systems:** Employed for\nburning off excess hydrocarbons to minimize environmental impact. #### 4. Energy Integration and Optimization To\noptimize energy use, heat integration is crucial. - **Heat Exchangers:** Used to recover heat from reactor effluents to\npreheat incoming feedstock, improving energy efficiency. - **Waste Heat Recovery:** Heat from reactors and distillation\ncolumns is utilized to generate steam for other parts of the process or for power generation. - **Recycling Loops:**\nUnreacted feed and by-products are recycled back into the process to maximize raw material utilization and minimize\nwaste. #### 5. Safety Measures Given the hazardous nature of the chemicals involved, several safety measures are\nimplemented: - **Material Safety Data Sheets (MSDS):** For all chemicals used in the process. - **Explosion-Proof\nEquipment:** Reactors and storage tanks designed to withstand high pressures and temperatures. - **Ventilation\nSystems:** To prevent the accumulation of flammable gases. - **Emergency Shutdown Systems:** Automated systems\nto safely shut down operations in case of an emergency. - **Personal Protective Equipment (PPE):** Required for all\npersonnel working in the facility. - **Leak Detection and Containment Systems:** To prevent the release of hazardous\nsubstances. - **Emergency Response Plans:** Established to address potential incidents, such as fires, explosions, or\nchemical spills. ### Conclusion The industrial synthesis of 1,3-butadiene involves a complex interplay of chemical\nreactions and requires careful consideration of reactor design, operating conditions, purification processes, and safety\nmeasures. By optimizing these factors, manufacturers can efficiently produce high-purity butadiene while minimizing\nenvironmental impact and ensuring worker safety, making the process suitable for large-scale industrial applications.\n\n"
**"Response":** "The operating conditions for the oxidative dehydrogenation of n-Butene in the synthesis of 1,3-Butadiene are as follows: \n\n- Temperature: 500-600 degrees Celsius\n- Pressure: 1-5 bar"

At step 216 of the method 200, the one or more hardware processors 104 of the system 100 are configured to employ the plurality of factual IR pairs generated at step 204, the plurality of DPO IR pairs generated at step 206, the plurality of synDIP IR pairs generated at step 208, the plurality of Logicore IR pairs generated at step 210, the one or more local RAIT IR pairs generated at step 212, and the one or more global RAIT IR pairs generated at step 214 of the method 200, forms the synthetic dataset which is used to generate a textual description of process engineering schematics of a chemical of interest.

In an embodiment, a process engineering schematics generation model is constructed by training the data-driven models such as AI models, and GEN AI models including but are not limited to the LLMs and SLMs or a combination thereof, with the synthetic dataset of the present disclosure. Then, the constructed process engineering schematics generation model is employed to generate the textual description of process engineering schematics such as the PFDs and the PIDs for the chemical of interest required by a user.

The methods and systems of the present disclosure generate the synthetic data from the domain knowledge of chemical process industry through the physics-aware GEN-AI enriched framework. The synthetic data generated by the methods and systems of the present disclosure is distinct, interdependent, and designed to systematically teach the data-driven models with different skills for understanding and working with the PFDs and the PIDs, and addresses the scarcity of specialized training datasets for these purposes. For example, the factual IR dataset is for facts, the SynDIP dataset is for descriptions, the Logicore dataset is for reasoning, the DPO dataset is for preference decisions, and the RAIT dataset is for grounded generation. Thus, the synthetically generated datasets of the present disclosure are in a structured format (e.g., QA pairs, IR pairs, text-based process descriptions), making them suitable for effectively training the data-driven models such as AI and GEN AI models.

Unlike simplified public PFDs and PIDs, the synthetic dataset of the present disclosure, particularly the SynDIP dataset, are designed to be comprehensive, detailed, and reflective of industrial-scale process complexities and modern engineering standards, providing richer and better training material. It bypasses the need for confidential industrial diagrams, for robust model training.

### Example Scenario:

The synthetic data of the factual IR dataset, the Direct Preference Optimization (DPO) dataset, the process synthesis, PFD and PID description (SynDIP) dataset, the Logicore dataset, the local RAIT dataset, and the global RAIT dataset generated by the methods and systems of the present disclosure is evaluated using a NVIDIA Nemotron-4-340B reward model to check the accuracy and effectiveness.

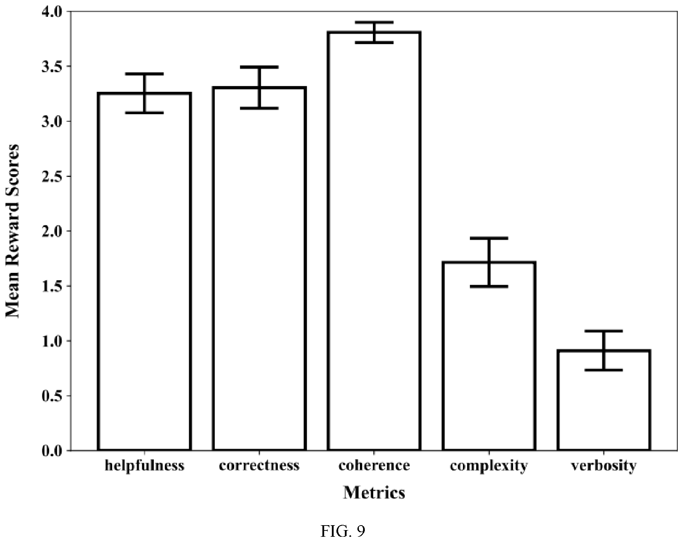

FIG. 9 is a graph showing evaluation results for the factual IR dataset, in accordance with some embodiments of the present disclosure. As shown in FIG. 9, each factual IR pair present in the factual IR dataset was scored on a 0-4 scale across five quality dimensions: helpfulness, correctness, coherence, complexity, and verbosity, ensuring high-quality data for instruction tuning. The plot shows strong scores in helpfulness, correctness, and coherence, indicating well-formed answers, while lower scores in complexity and verbosity suggest simpler, concise responses.

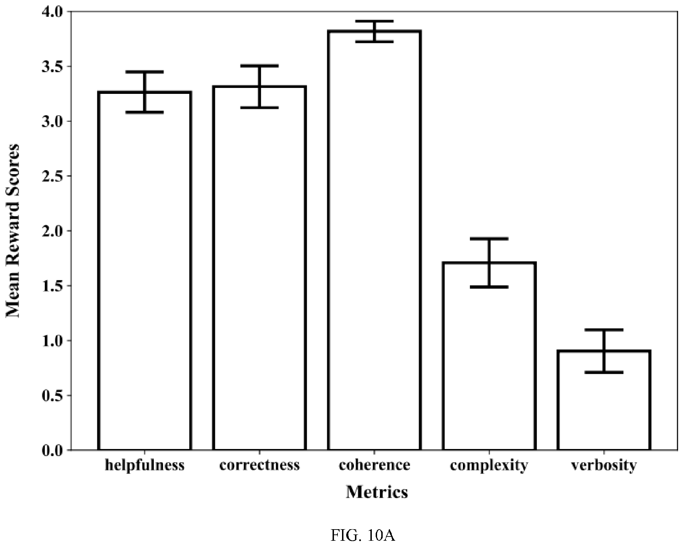

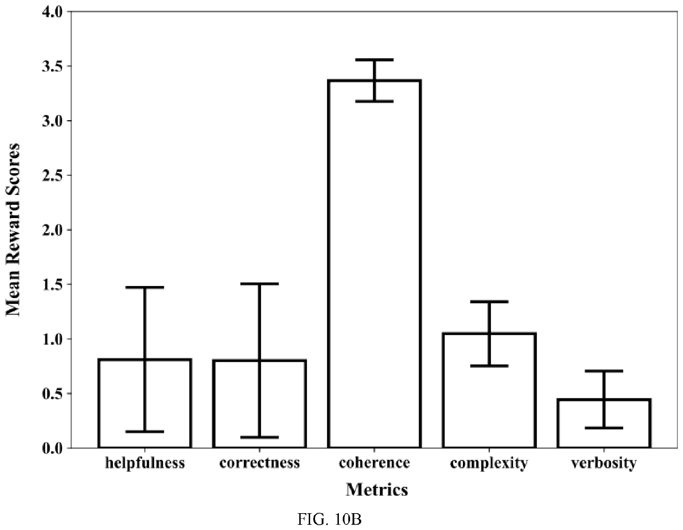

FIGS. 10A and 10B are graphs showing evaluation results for preferred candidate responses and dispreferred candidate responses of a DPO dataset, respectively, in accordance with some embodiments of the present disclosure. As shown in FIG. 10A, the high-scoring responses, assessed across multiple quality dimensions, serve as positive examples for fine-tuning models to produce human-preferred output. The plot highlights strong performance in coherence, correctness, and helpfulness, while scores for complexity and verbosity are comparatively lower, indicating simpler responses. As shown in FIG. 10B, the lower-scoring responses across multiple metrics serve as negative examples during preference optimization training. The plot reveals that while coherence remains relatively strong, the rejected responses perform poorly on helpfulness, correctness, complexity, and verbosity, highlighting major deficiencies in both informational value and expression quality.

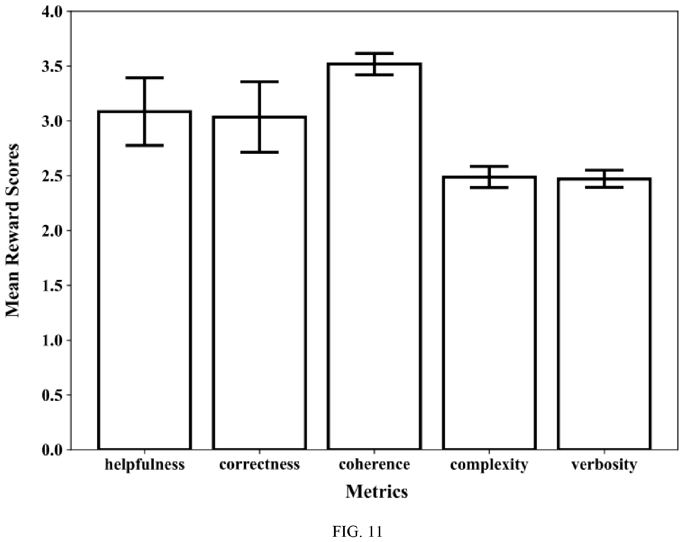

FIG. 11 is a graph showing evaluation results for the SynDIP dataset, in accordance with some embodiments of the present disclosure. As shown in FIG. 10, each chemical process sequence (process synthesis - PFD → PID descriptions) was scored across five key dimensions: helpfulness, correctness, coherence, complexity, and verbosity, validating the alignment with actual process schematics. The scores reveal strong performance in coherence and overall informativeness, while slightly lower scores in complexity and verbosity indicate that the descriptions are well-structured but may lean toward simpler, more concise phrasing.

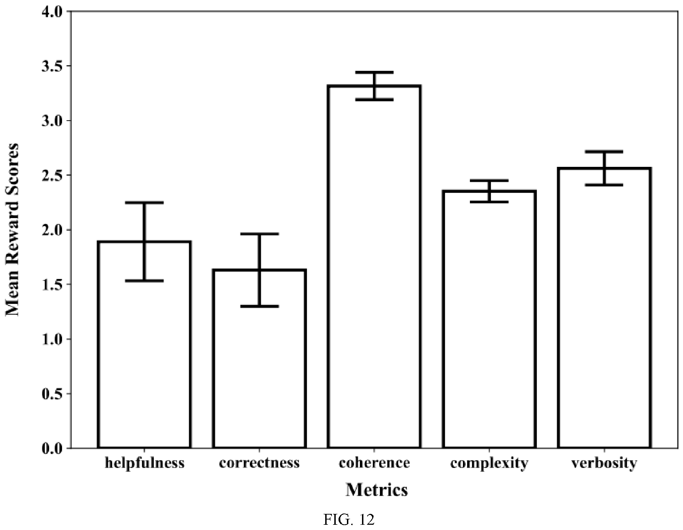

FIG. 12 is a graph showing evaluation results for the Logicore dataset, in accordance with some embodiments of the present disclosure. As shown in FIG. 12, each multi-step response was scored across five dimensions (helpfulness, correctness, coherence, complexity, and verbosity) to ensure logical validity and faithful representation of PFD/PID schematics. The plot shows high coherence and moderate complexity/verbosity, suggesting that the dataset captures logical reasoning with reasonably detailed explanations, though helpfulness and correctness could be improved.

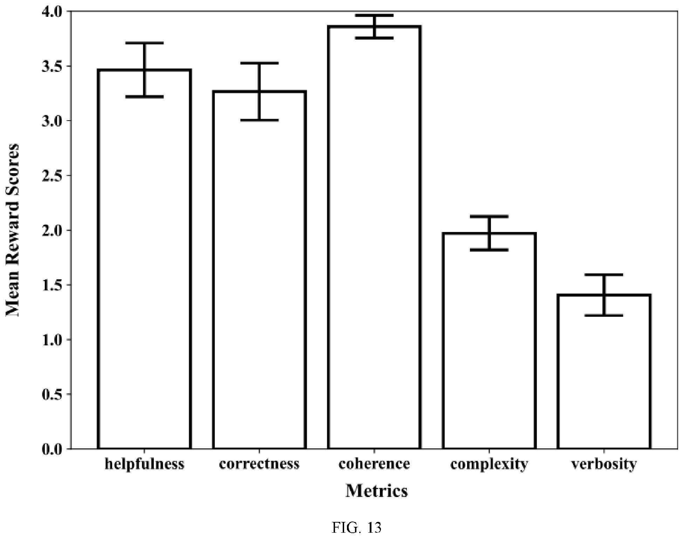

FIG. 13 is a graph showing evaluation results for the local RAIT dataset, in accordance with some embodiments of the present disclosure. As shown in FIG. 13, scores across five metrics (helpfulness, correctness, coherence, complexity, and verbosity) demonstrate the quality of retrieval-augmented IR pairs grounded in individual document chunks. The figure shows strong performance in coherence, correctness, and helpfulness, indicating the effectiveness of the retrieval-augmented strategy, though lower complexity and verbosity scores suggest answers are simpler and may lack elaboration.

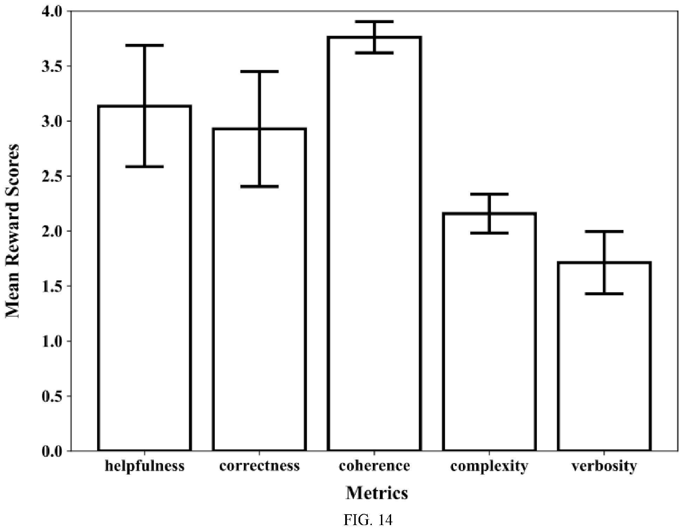

FIG. 14 is a graph showing evaluation results for the global RAIT dataset, in accordance with some embodiments of the present disclosure. As shown in FIG. 14, the scores reflect the effectiveness of both detailed and concise answers generated from clustered document chunks, demonstrating robust within-document and cross-document reasoning. The chart highlights strong coherence and helpfulness/correctness, affirming sound integration across multiple sources, while lower complexity and verbosity suggest that the responses prioritize conciseness over detailed elaboration.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problems of generating the synthetic data for process engineering schematics of a chemical of interest. The synthetic data is then used to auto-generate the PFDs and the PIDs for a new chemical, existing chemical, or a desired chemical of interest, using GEN AI models including but are not limited to large language models (LLMs) and small language models (SLMs).

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (200), comprising:
receiving, via one or more input/output (I/O) interfaces, (i) one or more topics, (ii) one or more seed data, and (iii) a plurality of known chemicals, from a repository, wherein the one or more topics are associated with a domain knowledge of a chemical process industry and the one or more seed data comprises (a) a set of annotated factual instruction-response (IR) pair examples, (b) a set of annotated preference IR pair examples, (c) a set of annotated chemical process blueprints, (d) a set of annotated process flow diagram (PFD) examples, (e) a set of annotated piping and instrumentation diagrams (PID) examples, (f) a set of annotated Logicore IR pair examples, (g) a set of annotated local Retrieval-Augmented Instruction-Tuning (RAIT) IR pair examples, and (h) a set of annotated global RAIT IR pair examples, associated with the domain knowledge of the chemical process industry (202);
generating, via one or more hardware processors, a plurality of factual IR pairs, from the one or more topics and the one or more seed data, using a first LLM and a multi-metric reward-based filtering, wherein the plurality of factual IR pairs captures domain-specific factual knowledge in the chemical process industry (204);
generating, via the one or more hardware processors, a plurality of direct preference optimization (DPO) IR pairs, from the one or more topics and the one or more seed data, using the first LLM and the multi-metric reward-based filtering, wherein each of the plurality of DPO IR pairs comprises a preferred candidate response and a dispreferred candidate response to an associated DPO instruction (206);
generating, via the one or more hardware processors, a plurality of synDIP IR pairs for each of the plurality of known chemicals, using the first LLM, a second LLM, the one or more seed data, and the multi-metric reward-based filtering, wherein each of the plurality of synDIP IR pairs comprises an industrial synthesis generation textual description, a PFD textual description, and a PID textual description, to an associated SynDIP instruction (208);
generating, via the one or more hardware processors, a plurality of Logicore IR pairs for each of the plurality of known chemicals, using the first LLM, the second LLM, the plurality of synDIP IR pairs, the set of annotated Logicore IR pair examples, and the multi-metric reward-based filtering, wherein each of the plurality of Logicore IR pairs comprises multi-step reasoning with explicit chain-of-thought explanations capturing procedural, logical and engineering response to an associated instruction (210);
generating, via the one or more hardware processors, one or more local Retrieval-Augmented Instruction-Tuning (RAIT) IR pairs for each of the plurality of known chemicals, using the first LLM, the second LLM, the plurality of synDIP IR pairs, the one or more seed data, the multi-metric reward-based filtering, and a Likert-scale-based filtering (212); and
generating, via the one or more hardware processors, one or more global RAIT IR pairs, using the first LLM, the second LLM, the plurality of synDIP IR pairs, the one or more seed data, a clustering technique, the multi-metric reward-based filtering, and the Likert-scale-based filtering, wherein the plurality of factual IR pairs, the plurality of DPO IR pairs, the plurality of synDIP IR pairs, the plurality of Logicore IR pairs, the one or more local RAIT IR pairs, and the one or more global RAIT IR pairs form a synthetic dataset (214).

2. The processor-implemented method (200) as claimed in claim 1, further comprising, employing, via the one or more hardware processors, the synthetic dataset, to generate a textual description of process engineering schematics of a chemical of interest (216).

3. The processor-implemented method (200) as claimed in claim 1, wherein:
generating the plurality of factual IR pairs from the one or more topics and the one or more seed data, using the first LLM and the multi-metric reward-based filtering, comprises:
decomposing the one or more topics, using the first LLM, to obtain one or more sub-topics for each of the one or more topics (204a);
generating a plurality of initial factual IR pairs, from each of the one or more sub-topics associated with each of the one or more topics, using the first LLM, and the set of annotated factual IR pair examples present in the one or more seed data (204b); and
applying the multi-metric reward-based filtering on the plurality of initial factual IR pairs, using a pre-defined factual threshold, to generate the plurality of factual IR pairs (204c), and
generating the plurality of DPO IR pairs from the one or more topics and the one or more seed data, using the first LLM and the multi-metric reward-based filtering, comprises:
decomposing the one or more topics, using the first LLMs, to obtain one or more sub-topics for each of the one or more topics (206a);
generating a plurality of initial DPO IR pairs, from each of the one or more sub-topics associated with each of the one or more topics, using the first LLMs and the set of annotated preference IR pair examples present in the one or more seed data, wherein each of the plurality of initial DPO IR pairs comprises a preferred candidate response and a dispreferred candidate response to the associated DPO instruction (206b); and
applying the multi-metric reward-based filtering on the plurality of initial DPO IR pairs, using a pre-defined preferred DPO threshold, and a pre-defined dispreferred DPO threshold, to generate the plurality of DPO IR pairs (206c).

4. The processor-implemented method (200) as claimed in claim 1, wherein generating the plurality of synDIP IR pairs for each of the plurality of known chemicals, using the first LLM, the second LLM, the one or more seed data, and the multi-metric reward-based filtering, comprises:
generating, for each of the plurality of known chemicals, a first industrial synthesis generation textual description and a second industrial synthesis generation textual description, for a predefined industrial synthesis generation template, using the first LLM and the second LLM, respectively, based on the set of annotated chemical process blueprints present in the one or more seed data (208a);
aggregating the first industrial synthesis generation textual description and the second industrial synthesis generation textual description, using one of the first LLM and the second LLM, to obtain an aggregated industrial synthesis generation textual description for each of the plurality of known chemicals (208b);
generating, for each of the plurality of known chemicals, a first PFD textual description and a second PFD textual description, for a PFD textual description template, based on the aggregated industrial synthesis generation textual description, using the first LLM and the second LLM, respectively, based on the set of annotated PFD examples present in the one or more seed data (208c);
aggregating the first PFD textual description and the second PFD textual description, using one of the first LLM and the second LLM, to obtain an aggregated PFD textual description for each of the plurality of known chemicals (208d);
generating, for each of the plurality of known chemicals, a first PID textual description and a second PID textual description, for a PID textual description template, based on the aggregated PFD textual description, using the first LLM and the second LLM, respectively, based on the set of annotated PID examples present in the one or more seed data (208e);
aggregating the first PID textual description and the second PID textual description, using one of the first LLM and the second LLM, to obtain an aggregated PID textual description for each of the plurality of known chemicals (208f);
forming, for each of the plurality of known chemicals, a plurality of initial synDIP IR pairs, using the aggregated industrial synthesis generation textual description, the aggregated PFD textual description, and the aggregated PID textual description (208g); and
applying the multi-metric reward-based filtering on the plurality of initial synDIP IR pairs, using a pre-defined synDIP threshold, to generate the plurality of synDIP IR pairs for each of the plurality of known chemicals (208h).

5. The processor-implemented method (200) as claimed in claim 1, wherein generating the plurality of Logicore IR pairs for each of the plurality of known chemicals, using the first LLM, the second LLM, the plurality of synDIP IR pairs, the set of annotated Logicore IR pair examples, and the multi-metric reward-based filtering, comprises:
generating, for each of the plurality of known chemicals, a plurality of aggregated Logicore instructions for each of the plurality of synDIP IR pairs, using the first LLM and the second LLM (210a);
generating, for each of the plurality of known chemicals, an aggregated Logicore response to each of plurality of aggregated Logicore instructions, using the first LLM, the second LLM, and the set of annotated Logicore IR pair examples (210b);
forming a plurality of initial Logicore IR pairs for each of the plurality of known chemicals, based on the aggregated Logicore response to each of plurality of aggregated Logicore instructions, from the plurality of synDIP IR pairs (210c); and
applying the multi-metric reward-based filtering on the plurality of initial Logicore IR pairs, using a pre-defined Logicore threshold, to generate the plurality of Logicore IR pairs for each of the plurality of known chemicals (210d).

6. The processor-implemented method (200) as claimed in claim 1, wherein generating the one or more local RAIT IR pairs for each of the plurality of known chemicals, using the first LLM, the second LLM, the plurality of synDIP IR pairs, the one or more seed data, the multi-metric reward-based filtering, and the Likert-scale-based filtering, comprises:
generating a synDIP instruction-response (IR) document for each of the plurality of known chemicals, from the plurality of synDIP IR pairs (212a);
extracting a plurality of text chunks for each of the plurality of known chemicals, by parsing the synDIP IR document (212b);
generating one or more initial local RAIT IR pairs from each of the plurality of text chunks, using the first LLM, based on the set of annotated local RAIT IR pair examples present in the one or more seed data, for each of the plurality of known chemicals (212c); and
applying the multi-metric reward-based filtering and the Likert-scale-based filtering on the one or more initial local RAIT IR pairs generated for each of the plurality of text chunks, using a predefined local RAIT threshold, the second LLM, and a predefined first Likert-scale threshold, to generate the one or more local RAIT IR pairs for each of the plurality of known chemicals (212d).

7. The processor-implemented method (200) as claimed in claim 1, wherein generating the one or more global RAIT IR pairs, using the first LLM, the second LLM, the plurality of synDIP IR pairs, the one or more seed data, the clustering technique, the multi-metric reward-based filtering, and the Likert-scale-based filtering, comprises:
generating a synDIP instruction-response (IR) document for each of the plurality of known chemicals, from the plurality of synDIP IR pairs (214a);
extracting a text for each of the plurality of known chemicals, from the synDIP IR document (214b);
generating one or more clustered synDIP IR documents, based on the text associated with each of the plurality of known chemicals, using a cosine similarity score (214c);
generating one or more initial global RAIT IR pairs from each of the one or more clustered synDIP IR documents, using the first LLM, based on the set of annotated global RAIT IR pair examples present in the one or more seed data (214d); and
applying the multi-metric reward-based filtering and the Likert-scale-based filtering on the one or more initial global RAIT IR pairs generated for each of the one or more clustered synDIP IR documents, using a predefined global RAIT threshold, the second LLM, and a predefined second Likert-scale threshold, to generate the one or more global RAIT IR pairs (214e).

8. A system (100), comprising:
a memory (102) storing instructions;
one or more input/output (I/O) interfaces (106); and
one or more hardware processors (104) coupled to the memory (102) via the one or more I/O interfaces (106), wherein the one or more hardware processors (104) are configured by the instructions to:
receive, (i) one or more topics, (ii) one or more seed data, and (iii) a plurality of known chemicals, from a repository, wherein the one or more topics are associated with a domain knowledge of a chemical process industry and the one or more seed data comprises (a) a set of annotated factual instruction-response (IR) pair examples, (b) a set of annotated preference IR pair examples, (c) a set of annotated chemical process blueprints, (d) a set of annotated process flow diagram (PFD) examples, (e) a set of annotated piping and instrumentation diagrams (PID) examples, (f) a set of annotated Logicore IR pair examples, (g) a set of annotated local Retrieval-Augmented Instruction-Tuning (RAIT) IR pair examples, and (h) a set of annotated global RAIT IR pair examples, associated with the domain knowledge of the chemical process industry;
generate a plurality of factual IR pairs, from the one or more topics and the one or more seed data, using a first LLM and a multi-metric reward-based filtering, wherein the plurality of factual IR pairs captures domain-specific factual knowledge in the chemical process industry;
generate a plurality of direct preference optimization (DPO) IR pairs, from the one or more topics and the one or more seed data, using the first LLM and the multi-metric reward-based filtering, wherein each of the plurality of DPO IR pairs comprises a preferred candidate response and a dispreferred candidate response to an associated DPO instruction;
generate a plurality of synDIP IR pairs for each of the plurality of known chemicals, using the first LLM, a second LLM, the one or more seed data, and the multi-metric reward-based filtering, wherein each of the plurality of synDIP IR pairs comprises an industrial synthesis generation textual description, a PFD textual description, and a PID textual description, to an associated SynDIP instruction;
generate a plurality of Logicore IR pairs for each of the plurality of known chemicals, using the first LLM, the second LLM, the plurality of synDIP IR pairs, the set of annotated Logicore IR pair examples, and the multi-metric reward-based filtering, wherein each of the plurality of Logicore IR pairs comprises multi-step reasoning with explicit chain-of-thought explanations capturing procedural, logical and engineering response to an associated instruction;
generate one or more local Retrieval-Augmented Instruction-Tuning (RAIT) IR pairs for each of the plurality of known chemicals, using the first LLM, the second LLM, the plurality of synDIP IR pairs, the one or more seed data, the multi-metric reward-based filtering, and a Likert-scale-based filtering; and
generate one or more global RAIT IR pairs, using the first LLM, the second LLM, the plurality of synDIP IR pairs, the one or more seed data, a clustering technique, the multi-metric reward-based filtering, and the Likert-scale-based filtering, wherein the plurality of factual IR pairs, the plurality of DPO IR pairs, the plurality of synDIP IR pairs, the plurality of Logicore IR pairs, the one or more local RAIT IR pairs, and the one or more global RAIT IR pairs form a synthetic dataset.

9. The system (100) as claimed in claim 8, wherein the one or more hardware processors (104) are further configured to employ the synthetic dataset to generate a textual description of process engineering schematics of a chemical of interest.

10. The system (100) as claimed in claim 8, wherein the one or more hardware processors (104) are configured to:
generate the plurality of factual IR pairs from the one or more topics and the one or more seed data, using the first LLM and the multi-metric reward-based filtering, by:
decomposing the one or more topics, using the first LLM, to obtain one or more sub-topics for each of the one or more topics;
generating a plurality of initial factual IR pairs, from each of the one or more sub-topics associated with each of the one or more topics, using the first LLM, and the set of annotated factual IR pair examples present in the one or more seed data; and
applying the multi-metric reward-based filtering on the plurality of initial factual IR pairs, using a pre-defined factual threshold, to generate the plurality of factual IR pairs, and
generate the plurality of DPO IR pairs from the one or more topics and the one or more seed data, using the first LLM and the multi-metric reward-based filtering, by:
decomposing the one or more topics, using the first LLM, to obtain one or more sub-topics for each of the one or more topics;
generating a plurality of initial DPO IR pairs, from each of the one or more sub-topics associated with each of the one or more topics, using the first LLM and the set of annotated preference IR pair examples present in the one or more seed data, wherein each of the plurality of initial DPO IR pairs comprises a preferred candidate response and a dispreferred candidate response to the associated DPO instruction; and
applying the multi-metric reward-based filtering on the plurality of initial DPO IR pairs, using a pre-defined preferred DPO threshold, and a pre-defined dispreferred DPO threshold, to generate the plurality of DPO IR pairs.

11. The system (100) as claimed in claim 8, wherein the one or more hardware processors (104) are configured to generate the plurality of synDIP IR pairs for each of the plurality of known chemicals, using the first LLM, the second LLM, the one or more seed data, and the multi-metric reward-based filtering, by:
generating, for each of the plurality of known chemicals, a first industrial synthesis generation textual description and a second industrial synthesis generation textual description, for a predefined industrial synthesis generation template, using the first LLM and the second LLM, respectively, based on the set of annotated chemical process blueprints present in the one or more seed data;
aggregating the first industrial synthesis generation textual description and the second industrial synthesis generation textual description, using one of the first LLM and the second LLM, to obtain an aggregated industrial synthesis generation textual description for each of the plurality of known chemicals;
generating, for each of the plurality of known chemicals, a first PFD textual description and a second PFD textual description, for a PFD textual description template, based on the aggregated industrial synthesis generation textual description, using the first LLM and the second LLM, respectively, based on the set of annotated PFD examples present in the one or more seed data;
aggregating the first PFD textual description and the second PFD textual description, using one of the first LLM and the second LLM, to obtain an aggregated PFD textual description for each of the plurality of known chemicals;
generating, for each of the plurality of known chemicals, a first PID textual description and a second PID textual description, for a PID textual description template, based on the aggregated PFD textual description, using the first LLM and the second LLM, respectively, based on the set of annotated PID examples present in the one or more seed data;
aggregating the first PID textual description and the second PID textual description, using one of the first LLM and the second LLM, to obtain an aggregated PID textual description for each of the plurality of known chemicals;
forming, for each of the plurality of known chemicals, a plurality of initial synDIP IR pairs, using the aggregated industrial synthesis generation textual description, the aggregated PFD textual description, and the aggregated PID textual description; and
applying the multi-metric reward-based filtering on the plurality of initial synDIP IR pairs, using a pre-defined synDIP threshold, to generate the plurality of synDIP IR pairs for each of the plurality of known chemicals.

12. The system (100) as claimed in claim 8, wherein the one or more hardware processors (104) are configured to generating the plurality of Logicore IR pairs for each of the plurality of known chemicals, using the first LLM, the second LLM, the plurality of synDIP IR pairs, the set of annotated Logicore IR pair examples, and the multi-metric reward-based filtering, by:
generating, for each of the plurality of known chemicals, a plurality of aggregated Logicore instructions for each of the plurality of synDIP IR pairs, using the first LLM and the second LLM;
generating, for each of the plurality of known chemicals, an aggregated Logicore response to each of plurality of aggregated Logicore instructions, using the first LLM, the second LLM, and the set of annotated Logicore IR pair examples;
forming a plurality of initial Logicore IR pairs for each of the plurality of known chemicals, based on the aggregated Logicore response to each of plurality of aggregated Logicore instructions, from the plurality of synDIP IR pairs; and
applying the multi-metric reward-based filtering on the plurality of initial Logicore IR pairs, using a pre-defined Logicore threshold, to generate the plurality of Logicore IR pairs for each of the plurality of known chemicals.

13. The system (100) as claimed in claim 8, wherein the one or more hardware processors (104) are configured by the instructions to generate the one or more local RAIT IR pairs for each of the plurality of known chemicals, using the first LLM, the second LLM, the plurality of synDIP IR pairs, the one or more seed data, the multi-metric reward-based filtering, and the Likert-scale-based filtering, by:
generating a synDIP instruction-response (IR) document for each of the plurality of known chemicals, from the plurality of synDIP IR pairs;
extracting a plurality of text chunks for each of the plurality of known chemicals, by parsing the synDIP IR document;
generating one or more initial local RAIT IR pairs from each of the plurality of text chunks, using the first LLM, based on the set of annotated local RAIT IR pair examples present in the one or more seed data, for each of the plurality of known chemicals; and
applying the multi-metric reward-based filtering and the Likert-scale-based filtering on the one or more initial local RAIT IR pairs generated for each of the plurality of text chunks, using a predefined local RAIT threshold, the second LLM, and a predefined first Likert-scale threshold, to generate the one or more local RAIT IR pairs for each of the plurality of known chemicals.

14. The system (100) as claimed in claim 8, wherein the one or more hardware processors (104) are configured to generate the one or more global RAIT IR pairs, using the first LLM, the second LLM, the plurality of synDIP IR pairs, the one or more seed data, the clustering technique, the multi-metric reward-based filtering, and the Likert-scale-based filtering, by:
generating a synDIP IR document for each of the plurality of known chemicals, from the plurality of synDIP IR pairs;
extracting a text for each of the plurality of known chemicals, from the synDIP IR document;
generating one or more clustered synDIP IR documents, based on the text associated with each of the plurality of known chemicals, using a cosine similarity score;
generating one or more initial global RAIT IR pairs from each of the one or more clustered synDIP IR documents, using the first LLM, based on the set of annotated global RAIT IR pair examples present in the one or more seed data; and
applying the multi-metric reward-based filtering and the Likert-scale-based filtering on the one or more initial global RAIT IR pairs generated for each of the one or more clustered synDIP IR documents, using a predefined global RAIT threshold, the second LLM, and a predefined second Likert-scale threshold, to generate the one or more global RAIT IR pairs.

15. One or more non-transitory machine-readable information storage mediums comprising one or more instructions which when executed by one or more hardware processors cause:
receiving, via one or more input/output (I/O) interfaces, (i) one or more topics, (ii) one or more seed data, and (iii) a plurality of known chemicals, from a repository, wherein the one or more topics are associated with a domain knowledge of a chemical process industry and the one or more seed data comprises (a) a set of annotated factual instruction-response (IR) pair examples, (b) a set of annotated preference IR pair examples, (c) a set of annotated chemical process blueprints, (d) a set of annotated process flow diagram (PFD) examples, (e) a set of annotated piping and instrumentation diagrams (PID) examples, (f) a set of annotated Logicore IR pair examples, (g) a set of annotated local Retrieval-Augmented Instruction-Tuning (RAIT) IR pair examples, and (h) a set of annotated global RAIT IR pair examples, associated with the domain knowledge of the chemical process industry;
generating a plurality of factual IR pairs, from the one or more topics and the one or more seed data, using a first LLM and a multi-metric reward-based filtering, wherein the plurality of factual IR pairs captures domain-specific factual knowledge in the chemical process industry ;
generating a plurality of direct preference optimization (DPO) IR pairs, from the one or more topics and the one or more seed data, using the first LLM and the multi-metric reward-based filtering, wherein each of the plurality of DPO IR pairs comprises a preferred candidate response and a dispreferred candidate response to an associated DPO instruction ;
generating a plurality of synDIP IR pairs for each of the plurality of known chemicals, using the first LLM, a second LLM, the one or more seed data, and the multi-metric reward-based filtering, wherein each of the plurality of synDIP IR pairs comprises an industrial synthesis generation textual description, a PFD textual description, and a PID textual description, to an associated SynDIP instruction;
generating a plurality of Logicore IR pairs for each of the plurality of known chemicals, using the first LLM, the second LLM, the plurality of synDIP IR pairs, the set of annotated Logicore IR pair examples, and the multi-metric reward-based filtering, wherein each of the plurality of Logicore IR pairs comprises multi-step reasoning with explicit chain-of-thought explanations capturing procedural, logical and engineering response to an associated instruction;
generating one or more local Retrieval-Augmented Instruction-Tuning (RAIT) IR pairs for each of the plurality of known chemicals, using the first LLM, the second LLM, the plurality of synDIP IR pairs, the one or more seed data, the multi-metric reward-based filtering, and a Likert-scale-based filtering;
generating one or more global RAIT IR pairs, using the first LLM, the second LLM, the plurality of synDIP IR pairs, the one or more seed data, a clustering technique, the multi-metric reward-based filtering, and the Likert-scale-based filtering, wherein the plurality of factual IR pairs, the plurality of DPO IR pairs, the plurality of synDIP IR pairs, the plurality of Logicore IR pairs, the one or more local RAIT IR pairs, and the one or more global RAIT IR pairs form a synthetic dataset; and
employing the synthetic dataset, to generate a textual description of process engineering schematics of a chemical of interest.
